# EUROPEAN PATENT APPLICATION

(11) **EP 4 835 085 A1**
(43) Date of publication of application: **04.11.2026**
(21) Application number: 24913389.3
(22) Date of filing: 26.12.2024
(51) Int. Cl.: C12N 15/81, C12N 9/10, C12N 9/80, C12N 9/12, C12P 19/30

(54) **MUTANT MICROORGANISM WITH ENHANCED ABILITY TO PRODUCE NICOTINAMIDE MONONUCLEOTIDE AND METHOD FOR PRODUCING NICOTINAMIDE MONONUCLEOTIDE USING SAME**

(30) Priority: 28.12.2023 KR 20230194549; 24.12.2024 KR 20240195182
(71) Applicant: Daesang Corporation, Seoul 03130 (KR)
(72) Inventor: LEE, Hee Jin, Seoul 07789 (KR); KIM, Soo Rin, Daegu 41566 (KR); SI, Yeon Ji, Seoul 07789 (KR); YOON, Na Eun, Seoul 07789 (KR); KIM, Ju Eun, Seoul 07789 (KR); KIM, Ji Seok, Seoul 07789 (KR); KIM, Ryeong Eun, Daegu 41566 (KR); PARK, Su Jeong, Daegu 41566 (KR); KIM, Sung Hee, Daegu 41566 (KR); LEE, Dong Ha, Daegu 41566 (KR)
(74) Representative: Ipsilon
(86) International application number: PCT/KR2024/021098
(87) International publication number: WO 2025/143782

(57) **Abstract**

The present invention relates to a mutant microorganism having enhanced nicotinamide mononucleotide productivity and to a method for producing nicotinamide mononucleotide using the same, wherein the mutant microorganism can produce nicotinamide mononucleotide in high yield by enhancing, attenuating, or suppressing the expression of enzymes, such as nicotinate phosphoribosyltransferase, that are involved in the nicotinamide mononucleotide production pathway.

## Description

### Technical Field

The present invention relates to a mutant microorganism having enhanced nicotinamide mononucleotide productivity and to a method for producing nicotinamide mononucleotide using the same.

### Background Art

Population aging is progressing rapidly worldwide, and it is expected that by 2060 the elderly population aged 65 or older will approach 41% of the total population. As the elderly population increases, the prevalence of age-related diseases is also increasing, thereby intensifying the socioeconomic and medical burden.

Nicotinamide adenine dinucleotide (NAD) is an essential coenzyme involved in cellular metabolism that receives hydrogen from various substrates and dehydrogenases to form NADH₂; NAD⁺ is the oxidized form of NAD and NADH is the reduced form of NAD. NAD⁺ plays a role in activating sirtuin gene, which is known as a longevity gene. However, the level of NAD⁺ in the body decreases with aging due to increased consumption of NAD⁺, and after approximately 60 years of age, the NAD⁺ level is reduced to about one-half of that. Because depletion of NAD⁺ can underlie not only aging but also diseases such as metabolic disorders, cancer, and neurodegenerative diseases, increasing NAD⁺ levels can ameliorate related deficiencies. Methods proposed for increasing NAD⁺ levels in the body include exercise, dietary control, maintaining a healthy diet, and NAD⁺ intake; however, because orally administered NAD⁺ is broken down in the digestive tract, it is more efficient to take it in the form of an NAD⁺ precursor.

Nicotinamide mononucleotide (NMN) is a type of nucleic acid that is a precursor of NAD+ and a physically stable compound. Recent studies have shown that supplementation with NMN enhances NAD+ biosynthesis and ameliorates various age-related symptoms such as Alzheimer's disease, diabetes, and vascular dysfunction.

NMN is mainly produced commercially using enzymes or yeast.

In enzyme-based methods, NMN is produced using phosphoribosylpyrophosphate synthase (PRS), which is involved in producing phosphoribosyl pyrophosphate (PRPP) - a precursor of NMN - in order to increase the expression level of PRPP, or using nicotinamide phosphoribosyltransferase (NAMPT), which produces NMN using nicotinamide (NAM) as a substrate; NMN production can be enhanced by using mutant forms of these enzymes with improved activity. In addition, NAM is converted to NMN using a protein mixture solution containing several enzymes such as NAMPT, PRS, and phosphoribulokinase (PRK).

In yeast-based methods, *Aspergillus*, which is recognized as safe, is used; NAD⁺ is converted to NMN as a substrate via pyrophosphatase, thereby producing NMN that can also be applied to foods.

Recently, methods have been developed for increasing the amount of NMN by inhibiting or enhancing the activity of various enzymes involved in producing precursors such as NMN using yeast, and further research is still needed in this area.

### [Prior Art Documents]

### [Patent Documents]

Korean Patent Publication No. 10-2021-0091255
Korean Patent Publication No. 10-2020-0061374

### DISCLOSURE OF THE INVENTION

### Technical Problem

An object of the present invention is to provide a mutant microorganism having enhanced nicotinamide mononucleotide productivity.

Still another object of the present invention is to provide a method for producing nicotinamide mononucleotide using the mutant microorganism.

### Technical Solution

One aspect of the present invention provides a mutant microorganism having enhanced nicotinamide mononucleotide productivity, in which the activity of nicotinate phosphoribosyltransferase is enhanced.

As used herein, "nicotinate phosphoribosyltransferase (NPT)" is an enzyme known to be present in bacteria, yeast, fungi, plants, insects, mammals, and the like, and to convert nicotinic acid (NA) into nicotinate mononucleotide (NaMN). Since nicotinic acid (NA) is structurally very similar to nicotinamide (NAM), the present invention suggests that nicotinate phosphoribosyltransferase (NPT) would have an enzymatic activity similar to that of nicotinamide phosphoribosyltransferase (NAMPT), which produces NMN using nicotinamide (NAM) as a substrate.

The nicotinate phosphoribosyltransferase (NPT) of the present invention may be, but is not limited to, a polypeptide having nicotinate phosphoribosyltransferase activity that converts nicotinic acid (NA) into nicotinate mononucleotide (NaMN). Nucleic acid and protein sequence information for the nicotinate phosphoribosyltransferase (NPT) and other enzymes involved in NMN biosynthesis can be obtained through known sequence databases (e.g., GenBank, UniProt).

According to one embodiment of the present invention, the nicotinate phosphoribosyltransferase (NPT) may be derived from one or more selected from the group consisting of *Saccharomyces cerevisiae*, *Schizosaccharomyces pombe*, *Methanosarcina mazei*, *Methanosarcina acetivorans*, *Desulforapulum autotrophicum*, *Methanococcoides burtonii*, *Chromohalobacter salexigens*, *Arabidopsis thaliana*, *Dictyostelium discoideum*, *Ciona intestinalis*, *Drosophila melanogaster*, *Ciona savignyi*, *Bacillus subtilis*, *Caenorhabditis elegans*, *Mycobacterium tuberculosis*, *Candida glabrata* or *Nakaseomyces glabratus*, *Kluyveromyces lactis*, *Eremothecium gossypii*, *Debaryomyces hansenii, Candida albicans, Yarrowia lipolytica, Aspergillus fumigatus, Neurospora crassa, Chaetomium globosum, Emericella nidulans, Aspergillus oryzae, Gibberella moniliformis, Cryptococcus neoformans*, and *Ustilago maydis*, but is not limited thereto.

The nicotinate phosphoribosyltransferase (NPT) includes a common motif regardless of origin. A motif is a small structural portion that appears in the amino acid sequences of various proteins, and can be divided into a structural motif, which forms a constant structure across proteins, and a sequence motif, which has a characteristic pattern in the amino acid sequence. Motifs commonly appear in proteins having a common function and are believed to contribute to functional expression.

The nicotinate phosphoribosyltransferase (NPT) of the present invention may include, based on structural homology or sequence homology, a motif consisting of 23 amino acids from positions 212 to 234 from the N-terminus of the amino acid sequence (hereinafter referred to as the '212-234 motif'). Among the residues of this motif, the histidine at position 232 (H232) has been reported to be autophosphorylated by ATP, thereby increasing catalytic efficiency (Biochemistry 35, 3917-3924; Biochemistry 37, 4189-4199). Accordingly, the 212-234 motif is expected to be a region related to the activity of nicotinate phosphoribosyltransferase (NPT), and a nicotinate phosphoribosyltransferase (NPT) including this motif can be expected to efficiently convert nicotinamide (NAM) into NMN.

According to one embodiment of the present invention, the nicotinate phosphoribosyltransferase (NPT) may include the above-described 212-234 motif.

Specifically, the nicotinate phosphoribosyltransferase (NPT) may be derived from the origins listed in Table 1 below, and the corresponding amino acid sequences and 212-234 motifs are shown in Table 2 below.

**[Table 1]**

| **No.** | **Origin** | **Gene** | **NCBI Accession No.** | **UniProt ID** |
|---|---|---|---|---|
| 1 | *Saccharomyces cerevisiae* | NPT1 | NP_014852 | P39683 |
| 2 | *Saccharomyces cerevisiae S288C* | NPT1 | CAA85352.1 | P39683 |
| 3 | *Candida glabrata* | - | XP_448893 | Q6FLK1 |
| 4 | *Kluyveromyces lactis* | - | XP_453357 | Q6CRT2 |
| 5 | *Eremothecium gossypii* | - | NP_983562 | Q75BW1 |
| 6 | *Candida albicans* | NPT1 | XP_889008/ | A0A1D8PRI0 |
| | | | XP_889008.1 | |
| 7 | *Debaryomyces hansenii* | | XP_462577 | Q6BGU4 |
| 8 | *Schizosaccharomyces pombe* | naprt | NP_001342932 | Q9UTK3 |
| 9 | *Yarrowia lipolytica* | - | XP_500338 | Q6CG74 |
| 10 | *Aspergillus oryzae* | - | BAE64333 | Q2U282 |
| 11 | *Chaetomium globosum* | - | EAQ93453.1 | Q2HDL6 |
| 12 | *Gibberella moniliformis* | NPT1 | AAN74808 | Q8J2R5 |
| 13 | *Neurospora crassa* | nic-6 | XP_965789 | Q7SI03 |
| 14 | *Aspergillus fumigatus* | - | XP_746744 | Q4WAN0 |
| 15 | *Emericella nidulans* | - | XP_682385 | Q5ARG4 |
| 16 | *Cryptococcus neoformans* | - | XP_568039/ | QSK8P0 |
| | | | XP_568039.1 | |
| 17 | *Cryptococcus neoformans* | - | EAL18922 | - |
| 18 | *Ustilago maydis 521* | - | XP_760597/ | - |
| | | | XP_760597.1 | |
| 19 | *Methanosarcina mazei* | pncB | WP_011034984 | Q8PSJ3 |
| 20 | *Desulforapulum autotrophicum* | pncB | WP_012662670 | C0QFM5 |
| 21 | *Methanosarcina acetivorans* | pncB | WP_011022501 | Q8TMW6 |
| 22 | *Methanococcoides burtonii* | pncB | WP_011498483 | Q12Z05 |
| 23 | *Chromohalobacter salexigens* | pncB | WP_011506244 | Q1QZ10 |
| 24 | *Dictyostelium discoideum* | naprt | XP_647373 | Q55G10 |
| 25 | *Arabidopsis thaliana* | NAPRT1 | NP_195412 | Q8RWM2 |
| 26 | *Ciona intestinalis* | - | NM_001032533 | F7APS6 |
| 27 | *Arabidopsis thaliana* | NAPRT2 | NP_179923 | Q84WV8 |
| 28 | *Ciona savignyi* | - | - | H2YXH9 |
| 29 | *Drosophila melanogaster* | Naprt | NP_001097077 | Q9VQX4 |
| 30 | *Drosophila melanogaster* | Naprt | NP_722961 | Q9VQX4 |
| 31 | *Bacillus subtilis* | pncB | WP_003228788 | O32090 |
| 32 | *Caenorhabditis elegans* | nprt-1 | NP_500265 | Q95XX1 |
| 33 | *Mycobacterium tuberculosis* | pncB2 | WP_003403007 | P9WJI7 |
| 34 | *Mycobacterium tuberculosis* | pncB 1 | WP_003911483 | P9WJI9 |

**[Table 2]**

| **No.** | **Amino acid Sequence** | **SEQ No./ Sequence homology** |
|---|---|---|
| 1 | MSEPVIKSLLDTDMYKITMHAAVFTNFPDVTVTYKYTNRSSQLTFN KEAINWLKEQFSYLGNLRFTEEEIEYLKQEIPYLPSAYIKYISSSNYK LHPEEQISFTSEEIEGKPTHYKLKILVSGSWKDTILYEIPLLSLISEAYF KFVDIDWDYENQLEQAEKKAETLFDNGIRFSEFGTRRRRSLKAQDL IMQGIMKAVNGNPDRNKSLLLGTSNILFAKKYGVKPIGTVAHEWV MGVASISEDYLHANKNAMDCWINTFGAKNAGLALTDTFGTDDFL KSFRPPYSDAYVGVRQDSGDPVEYTKKISHHYHDVLKLPKFSKIICY SDSLNVEKAITYSHAAKENGMLATFGIGTNFTNDFRKKSEPQVKSE PLNIVIKLLEVNGNHAIKISDNLGKNMGDPATVKRVKEELGYTERS WSGDNEAHRWT | 2/ 100% |
| 2 | MSEPVIKSLLDTDMYKITMHAAVFTNFPDVTVTYKYTNRSSQLTFN KEAINWLKEQFSYLGNLRFTEEEIEYLKQEIPYLPSAYIKYISSSNYK LHPEEQISFTSEEIEGKPTHYKLKILVSGSWKDTILMRSLTVLISEAY LIVTSTGLRNHRQAEKKAETLFDNGIRFRIHGTRRRRSLKAQDLIMQ | 3/ 94.19% |
| | GIMKAVNGNPDRNKSLLLGTSNILFAKKYGVKPIGTVAHEWVMGV ASISELDYLHANKNAMDCWINTFGAKNAGLALTDTFGTDDFLKSF RPPYSDAYVGVRQDSGDPVEYTKKISHHYHDVLKLPKFSKIICYSDS LNVEKAITYSHAAKENGMLATFGIGTNFTNDFRKKSEPQVKSEPLNI VIKLLEVNGNHAIKISDNLGKNMGDPATVKRVKEELGYTERSWSG DNEAHRWT | |
| 3 | MSSMTPAITSLLDTDMYKITMHAAVFTNFPDARVVYKFTNRTAQF HFNKRAVDWIKEQFRLLGDLTFTHDDVEYLAREIPFLPAKYLHYIE NGFTLKPDEQIELDCQEIKGKEDQYDLHILVKGLWIDTILYEIPILAL LSEAYFKFVDTDWDYENQLSNAKEKALRLFENNLSFSEFGTRRRRS FKTQDLVMEGIMQAVKENPEKYRPLLLGTSNILFAKKYGVKPIGTV AHEWIMGIASITGDYPETNRIAMDYWIKTFGKEHAGLALTDTFGTD DFLKSFKPPYSDYYIGVRQDSGDPIKYTEKIAHHFHDVLKLPKFSKFI CYSDSLNIDKAIEYGKVAEAHGIKSTFGIGTNFTNDFHKKSDPSVKS APLNIVIKLLEVNGNHSIKISDNAGKNMGDPDTVKKVKEQLGYVER QWAGGNEAHRSAA | 4/ 70.52% |
| 4 | MEPVITSFLDTDMYKLTMHAAVYTHFKDVKVKYKYTNRSPQMTF NKEAVEWLKGQFLLLANIRLTTEELKYIKEAIPYLPAEYLEFISNGG FEVNPKDEIKFEAREIEGEPGHYELDISIEGLWIKTIWYEIPVLALVSE AYFKFVDTDWTYDGQVEKAYAKAKKLLDHDIVFSEFGTRRRRSFK TQDLVMQGIINAAKDCDKSKYVLGTSNVLFAKKYNVNPIGTVAHE WMMGIASITNDYLNANKNAMDYWIETFGMENAGLALTDTFGTDS FLKSFYPPYSDAYIGVRQDSGDPILYTEKIAHHYNDVLKLPKFSKIIC YSDSLNPDRAIEYAQVAHKHGLKATFGIGTNFTNDFQRKSDVSVKS EPLNIVIKLLEVNGNHAIKISDNLGKNMGDPETVRRVKEELGYVER KYAGDNEAHRWAA | 5/ 69.63% |
| 5 | MTSEEAAIKSLLDTDMYKLTMHAAVYVNFPDTEVVYKYTNRSAG LSFNKAAIVWLEDQVSKLATLRFTAEEVAYLKETLPFLPAQYIDYIS SPECRMDPATQVQLLHEQREGEERYDLNITVTGLWKDTILYEIHML ALISEAYFKFVDTDWVLDGQVEQAYRKTRLLLDNGLVFSEFGTRR RRSLLVQDLVLQGIAEAVADSGTGDTQFIGTSNVYFAKKYGVKPV GTVAHEWYMGIAALTDDYRNANKNAMDFWLNTFGSEQAGLVLT DTFGTDTFLPMFRPPYSDVYDGVRQDSGDPAVFTEKVAHHYLNVL HYPRFSKVICYSDSLNPEKALQYAEVARAHGMRASFGIGTNFTNDF RRHSAPDAKSEPLNIVFKLLTVNGRPAIKISDDLGKTMGDPEKVEEV KRELGYIERTWEGSDEAHRWKD | 6/ 63.85% |
| 6 | MNGTEDKPVIKSFLDTDLYKLFMHAAVNKQFPDVPVKYRYTNRTP QLKLNSQAISWLKKQIEYLGDLRFSAEEILYLHRVLPQLPSDYLEYL ADFKLVPSSQIKYLDNDEGDFELEIVGKWNDTILYEIPLLALVSEAY FKFVDTDWNYEGQYALAQKKAKQLISNECNFSEFGTRRRRSYESQ EIVIKAIKDVQIDTQSKYIAGTSNVYFAMKYDLPPIGTVAHEWYMGI ASITQDYVHANKLAMDYWIDTFGAKYAGLALTDTFGTDNYLTMF VAPYVNEYSGVRQDSGDPELYAEKIARHYEKMGIAKNTKIICFSDS LNVEKCLKYKNTADKLGLISTFGIGTFFTNDFNKLSNGEKSQPMNIV IKIKEANGKPAIKISDNIGKNMGDQATVDRVKQELGYTERTWSEGD ETHRWSK | 7/ 58.31% |
| 7 | MTTDIPIITSILDTDLYKLTMHAAVVKHFPNIPVVYRYTNRTPSMVL NKEAIDWLKYQISKLSDLRVSSEELEYLRKALPQMPEVYLKYLETF QLFPDKQIKYFNDESNFEEFEIEMKGKWDETMLYEIPLLALISEAYF KFVDTDWNYDGQAERAEDKCAQLFKNECTFSEFGTRRRRSFKTQD LVVKNLCDFAGKNPDKKHFLGTSNVLLAKKYNTTPIGTVAHEWFM GIASITQDYTNANKLAMDYWLDTFGPEHAGLALTDTFGTDAYLKV FTKPYTDYYTGVRQDSGDPESFAEKLADHYKKQGYPDFSKIICFSD | 8/ 56.94% |
| | SLNVEKCLKYKQKVNSLGLIASFGIGTFFTNDFASVSDPSTKSVPLNI VIKLKEANGNPSIKISDNLGKNMGDPAVVSRVKKELNYEEHSWAE GDEEKRWD | |
| 8 | MSEPAVVSILDTDLYKLTMLQAVLEHYPDAQVSYKYTNRSPKMAL 9 NQEAYNWLREQIRGLRNLHLLPEEEQWLRKNCPYLKESFYEFMHE FEFDPENSISLNYDSETKDLSIFIHGLWKNTIFYEIPLLALVSESYFKF VDKDWSPEGQFEKAYEKGKRLIRAGCAFTDFGTRRRRDPHTQEIVL QGLMKAQEDFKGPGSFLGTSNVYFAAKYNLNVSGTVAHEWYMGI AAITQNYKQANRIASLKWVQTFGTSLLIALTDTFSTDVFLKSFTANS ADDLANVFHGVRQDSGCAEEYIEKVVKHYKSIGVDPSTKVIVHSD ALNVDRCIELYKYCEKCGIKSAFGIGTNLTSDFQKVSNPSEVSKPMN IVIKLFSAEGTKAVKISDDIMKNTGDRDAVIQAKHQLCLPIA | 9/ 45.67% |
| 9 | MSTITSLLDTDLYKLTMQAAVLQHFPAAQATFLFKNRTPSKQLNDD AIEWLKSEIAALGELRFTEDEIVFLQKHVGFLPAEYFEYLKTCQLDP AAQVKVTVNTEGHLEIEVNGPWKDTILYEIPLLALVSEAYFKFVDK DWSYDGQSELAATKAQELIAQGCAFSEFGTRRRRSLKTHDIVIAGIL EGLKSAQGNGIFTGTSNVYLAKKYNLKPIGTVAHEWMMGVAAAT GDYSTANLRAMELWIQTVGDANAGVALTDTFGTESFLLDFNKPLT DIYNGVRQDSGDPLEYTKLLGDHYKQLGYEPMSKVIVYSDSLDVE KCGKYKAAAAENGLKAAFGVGTFFTNDFKRLSDGQKSTPLNIVIKI QQLNGQSCIKLSDNLSKNMGDPETVERVKRELGYVEKGDVIDESK RWN | 10/ 53.97% |
| 10 | MDQEGSPPIPEGICSLLDTDLYKLTMQCAVLKYFPDTHVTYGFTNR TPHMKLTRGAHKWLLKQMDRLANIRITEEEIKFLKTRCPYFNDAYL DFLTTFKLKPSEQIEIKFTPVNDTGSDSDTGDVEYLVKGLWVDTILY EIPLLALTSEAYFMFSDKDWDYSCQEEKAYRKGCTLLENGCIFSEF GSRRRRDYHTHDLVMVGLMKAAEEGKRQGWKGRFTGSSNVHFA MKYGVDPVGTVAHEWYMTIAAITDDYENANELALRYWLGCFGKG VLGIALTDTFGTPAFLDAFRKPIPAFTSAGAGAVSTSASGPATTNEST VQSEAETKAPITAPLRDGGARTSHETYAQAYTGVRQDSGDPVYFV KMVRDFYDREGITDKKTMVFSDSLNIEHCLEYKVIAEEAGFQPVFG VGTFFTNDFTNKSNDEKSKPLNIVIKISTANGHPAVKLSDNMGKNT GDKQKVQEVKKKLGYVEHEWEEGDESNRWSKR | 11/ 44.38% |
| 11 | MDFNSSSPYPEGVISFLDTDLYKLTMQCAVLKYYKNVPVTYAFTN RTPEKKLSRKAFRWLEDQVRKLGNISLSAEELYYLKEHCPYLSPAY LEFLSEFRLRPREQVVLSFLPTGEDTGAESDIGDLDIKISGIWSEAILY EIPLLSLTSEAYFKFMEPDWTYEGQEDQAFEKGMRLLEAGCVFSEF GTRRRRDYHTQALVFRGLTKASKEAEKRGLSGKLSGTSNVHLAMR FNIPPVGTVAHEWFMGSAAIVGDYRKATEEALRHWVGCFGEGVLG IALTDTFGTPDFLTAFSKPIEYLEPPSPTTARKPSVADSFISTSPSVAS HQKPNKTYAEVFTGVRQDSGDPKTFVKIIGKFYREQGIKDKKVIVF SDSLNIDRCLEYKQVSEEAGFQPTFGVGTFLTNDFVNTKTGKKSTPL NIVIKLSSADGNPAIKISDNIGKNTGDKATVEKVKRELGYVEKMWE GGDETARWGREDDAPKQ | 12/ 43.87% |
| 12 | MDFNSSSPFPEGVISFLDTDLYKLTMQCAVFKFFKDVPVTYAYTNR TPDKKLSRTAFKWLEEQIRKLGNISLSTDEYLFLKKHCDYLSDDYL NFLKEFRLSPREQVVATFTPVGEDNGDDSIEGDVDIQIKGTWVDTIL YEIPMLALTSEAYFKFMDTDWNYEGQEKQAFEKGLKLLEAGCITSE FGTRRRRDYHTQALVFRGLVQASKEAEKKGFPGKLSGTSNVHLAM RFNIPPVGTVAHEWFMGVAAIIGDYRSATEVALRHWVACFGNKLG IALTDTFGTQEFLRAFTQPVQTIEGGFPAETFKKADGTMKTYAETFA GIRQDSGDPAEYTKWMKEFYDKQGITDKKLIVFSDSLNIERCLEYK | 13/ 43.79% |
| | KIAEDLGFQPTFGVGTYLTNDFVHLTTGKKSVPLNIVIKISSAAGRP AVKISDNIGKNTGDKETVEKVKQELGYVEREWKEGDETSRWGKE | |
| 13 | MDFNSSLPHPAGVISFLDTDLYKLTMQCAVLKYFKDVPVTYSFTNR TPEKKLSREAFVWLEEQVMKLGNISLSPEELQFLKTHCPYLTEEYL DYLSEFRLRPREQVAVSFRPDGDSDLGDIHYDIKGNWAETILYEIPL LALTSEAYFKFMDTDWDYDGQEEKAYEKGMRLLEAGCVFSEFGT RRRRDYHTQALVFRGLTKAAKEAEKRGLTGKLSGTSNVHLAMRF NIPPVGTVAHEWFMGNAAILGDYKSATEEALSRWVGCYGPGVLGI ALTDTFGTPEFLRAFSKPMSASGEPVPQPRDRKISTADAFISAAKDFI KDLHPDKTYAQVFTGVRQDSGDPKEFVKLMRKFYDEQGIKDKKVI VFSDSLDIDRCLEYKEVAEAAGFQPTFGVGTYFTNDFVHKATGKKS TPLNIVIKLSSAAGNPAVKISDNVGKNTGDKATVEKVKRELGYVEK DWSEGDESARWGHDGDAATA | 14/ 43.29% |
| 14 | MSQETTPPYPEGIFSLLDTDLYKLTMQCAILKYFPDVHVTYGFTNRT PDMKLTRGAYKWLLEQMDRLANVRVTDEEIAFLKKQCPYFNHAY LRYLSTFQLKPSEQIDIKFRPVQDSGSDDDLGDIEYMVKGLWVETIL YEIPLLALTSQAYFMFTDKDWDHSNQEEKAFRKGCTLLENGCIFSE FGSRRRRDYHTQDLVMQGLCRAAEEGKRQGWKGVFSGTSNVHFA MRYGVTPIGTVAHEWFMAIAAITDDYENANELALRYWLGCFGEGV LGIALTDTFGTPAFLDAFRKPIPHHTSAGVGAVATIASGASTTSESQP QTEAETKPPVTAPLHENDSQHSPKTYAQVYTGVRQDSGDPTYFVK MVRDFYDNEGIKEKKTVVFSDSLNIEHCLEYKTIAEEAGFAPIFGVG TFFTNDFTNKSDGKKSKPLNIVIKIATANGRPAVKLSDNMGKNTGD KNMVQEVKKRLGYIEHYWEEGDESNRWAKQG | 15/ 43.22% |
| 15 | MGEHSLLPDGVFSLLDTDLYKLTMQCAILKYFPDVQVTYGFTNRTP HMKLTRGAYKWMLAQMDKLANIRVTEDEIAFLKRRCPYFNTAYL DFLTNFRLKPSEQIDINFTPVNDTGSDSDFGDIDYIVKGAWVDTILY EIPLLALTSQAYFMFSDKDWNYECQEGKAYRKGYVLLENGCTFSE FGTRRRRSYHTQDLVMQGLCRAAREGKAKGLPGVFTGSSNVHFA MKYDVDPVGTVAHEWYMTIAAITDDYENANEMALKYWLGCFGE GVLGIALTDTFGTPAFLDAFRKPIPDYTSAGTGAVSTTASGPSTTAE SNIQSEAETKAPITAPLSPDHPPPAVKTYAQVYAGVRQDSGDPSYFV KMARDFYDREGITGTKTVVFSDSLDIEHCLEYKVLAEEAGFKPVFG VGTFFTNDFINTTTNEKSQPLNIVIKISSANGRAAVKLSDNMGKNTG DKDTVQAVKKRLGYVEHEWEEGDERNRWARK | 16/ 42.17% |
| 16 | MPDKLHLPIDDVQIPFSILDTDLYKLTMQNAVLHHFSDAHVVIKFT NRSPQMLFSKECFDWVQQRVNDLSKLKLTSEERKELSKACPYFSES YLDYLSNMQLDPVKQVKLTFIPQGSNEKGEKMGEIGCVIEGPWKD TMLYEVPVMAILSEGYFKFVDTDWDYDGQFELAKKKALDLLNPPA PTTSLSFSEFGTRRRRSFKAQDIIMRGLIAGHEEYKSKGGSQGILSGT SNVYLALKYGLNPVGTIAHEWIMAVGATYGYRGANGRAMDMWE EVYPPGTKFSSPLTMLTDTYTAAIFFKDFISDPARALRWAVLRQDSG DAFKFVEDAKEAWRTIEDKAGIKRDVGPNGEEEVAKGKKVIFSDG LDVEKAIKLQQGCDKAGMAASFGIGTDLTNDFRKASDPSQKSKAL NMVIKLNKINGKDCIKLSDDKGKHTGSLEEVRKAQQELGINKN | 17/ 40.50% |
| 17 | MPDKLHLPIDDVQIPFSILDTDLYKLTMQNAVLHHFSDAHVVIKFT NRSPQMLFSKECFDWVQQRVNDLSKLKLTSEERKELSKACPYFSES YLDYLSNMQLDPVKQVKLTFIPQGSNEKGEKMGEIGCVIEGPWKD TMLYEVPVMAILSEGYFKFVDTDWDYDGQFELAKKKALDLLNPPA PTTSLSFSEFGTRRRRSFKAQDIIMRGLIAGHEEYKSKGGSQGILSGT SNVYLALKYGLNPVGTIAHEWIMAVGATYGYRGANGRAMDMWE EVYPPGTKFSSPLTMLTDTYTAAIFFKDFISDPARALRWAVLRQDSG DAFKFVEDAKEAWRTIEDKAGIKRDVGPNGEEEVAKGKKVIFSDG | 18/ 40.50% |
| | LDVEKAIKLQQGCDKAGMAASFGIGTDLTNDFRKASDPSQKSKAL NMVIKLNKINGKDCIKLSDDKGKHTGSLEEVRKAQQELGIDKN | |
| 18 | MCAAPSTSAPVAKHASDASPIRSILDTDLYKLTMQQAVLRHYPHTR VAYKFTNRSAATMKFTRQAMDRIRNHIDNLVHLSLSAQERAWLER SCPYLGKDYLDYLEAFRFQPKQQVQLRFLPTGEDGWGHLDLNVSG VWSDVIFYEVPLMAIVSEVYFSTIDTDWSLQDQYQQAFDKACRLTS NGIRYSEFGTRRRRSYQTHRIVLQGLMAGDLSASSGCSSGKLLGTS NVHFAQQFDLVPIGTVAHEWTMAIAALQGYAHSNLKALQLWDAV YSAPDFVANSATHDLTIALTDTFSTNVFWNDLLDNPSGIEIARRWR GLRQDSGDSKAFAQKALDAYRSIGVDPKSKVVIYSDGLDVDRCLE LAAYSNHIGIGAAFGIGTSFTNDFIQLSTAQKSKPLNIVIKLDSVEHR RVVKISDDLTKNTGDPTEVLAVKRRFGIPTPSSATPADASVINHLDP GAPPNPSSHAHLGTDDATMTQVSPLDQRNVAAGVELTPPNEPRRLL SARSTSKLSSPKNVASSCAWSNCALASKLVSSIPAALIAGVIERARE GDCDCEPDAPREMCLALNAANGDDAGATAPIAGLNSEIGVPGVPG VPGEPIRLLLCANPLL | 19/ 39.25% |
| 19 | MIKSILDNDLYKFTMQMAVLELFPKAEAEYRFTNRGLQRFSREFVE ELRRVIDEEISGLRLTEEEYRWLGEKCPFLKPMYLEYLKNFRFKPEE VEICLTRENDLDMRIKGPWHSTILWEIVLMAAVSELYFTTIEKEWN GSTKNPGTPESATLESVLEAYGEKILEIGKVLEENGCLFSEFGTRRR RSFELHDQVMRSLVRIKTLTGTSNVYFAKKYGVKPIGTVGHEWIM GTSALVGLRYANRFAFENWVDVYNGDLGIALTDTFGSEAFFKDMD LKLSKIYDGFRHDSGDPYTFVDRVIEHYGKMGIDPMKKVIVFSDAL NAEAAVKLKKYCEGKINCSFGIGTSLTNNSEFFRESPPLNMVIKLHSI NGIPVVKLSDSPEKETGERDALRVANYIVGRKGLDE | 20/ 37.26% |
| 20 | MIQSILDNDLYKFTMQQAVHMLYPRVDVEYEFINRSNTPFPKDFAQ RLQVEVQGMKNFRLTPEEKEYLDKTCYFMTPVYLDFLEHYTFDPD EVTVSQTNSELSVTIKGPWYRTILWEVPLMAIISELYFVMTNARPLP DEQIRVINLNKAKILSCNNIRYADFGTRRRFSSSGHEALIRDILALEH NTLIGTSNVNLARLFNIKPIGTMAHEWIMFHGVLNGYRMANPTAV AAWATAFHGHLGIALTDTFTTDIFLSTFDTLHAKLFDGVRHDSGDPI AFIDRIVDHYKKLHIDPITKTIVFSDGLDIDKAVHIHNHCINRIRDSY GIGTNLTNDVGVTPLNMVIKLAKCRTAPEKDWHNAIKLSDDKGKH TGDSEELAHCIKVLERGM | 21/ 36.14% |
| 21 | MIKSILDNDLYKFTMQMAVLELFPKAEAEYRFTNRGSHHFSEEFVE KLRRVIDEDISALMLTEDEYQWLGENCSFLKPMYLEYLKNFRFKPG EVEVCLTEEKELDIRIKGPWHSTILWEIVLMAAVSELYFTTIEKEWN GKEWDGNISATSESILTAYGEKILEIGKILEENGCLFAEFGTRRRRSF ELHDQVMKTLLQIETLTGTSNVFFAKKYGLKPIGTVGHEWIMGTSA LIGLRYANRFAFENWVEVYKGDLGIALTDTFGSEAFFKDMDLKLSK IYDGFRHDSGDPFTFVDRVIDHYRKMGIDPMKKVIVFSDALNAEAA IRLKKYCQDKINCSFGIGTSLTNNSEFFRESPPLNMVIKLHSVNGIPV VKLSDSPEKETGERDALRVANYIVGRKGLDE | 22/ 35.41% |
| 22 | MIRSILDNDLYKFTMQMAVLELFPNARAEYRFINRGAQSFTNDFVN ELRRIINKDISKIALSEDEYIWLKDNCPFFKPSYIEYLKNFRFNPEEVK IVLTEDNELELCIEGPWHSSILWEIVLMSTISELYFTVTDNKGEEISAS NANDPENTLMEEYSSFIGDMGKELDAKGCIFSEFGTRRRRGFKLHD KVVEVLHELDSFSGTSNVYFAKKYGVRPIGTIGHEWIMGNSALVGL RNANKFAFDNWVKVYKGDLSIALSDTFGSKPFFQNFSIGLAKIYDG VRHDSGDPIKFADEVIEHYKKLGIDPMKKVLVFSDSLHVSDAVKLK EYCSGRINCSFGIGTTLTNNPDFFSYNPPLNMVIKLHKIDGIPVVKLS DSVEKATGDKDALRVANYIFGRKGLDEQF | 23/ 34.13% |
| 23 | MLDSLLDNDFYKFTMQSAVIKRFPYARARYAFINRGEHAFPPGFGE QLREAVDAMADLSLSVEEKRFLERTCPYLDPTYLDFLSGFRYDPSE VDIVQQGERFELRIEGLWYRTILWEVPLMALISEIWYRMGDGKRDA DAIIDMRTRDKIEHYKRLGLKIAEFGTRRRYSYDVHDRVVASLRHH GGDTFSGSSNVHLAMRHGVKPIGTHAHEWFMFHGARFGFKMANS LALEHWVDVYRGDLGIALTDTFTSATFFDSFDKKFAKLFDGVRHDS GDPLEFAAATIAHYERMGIDPRTKTIIFSDALTPERVERIDAFCRGRI TTAFGIGTNFTNDVGVTPMNMVIKMTEARPEGQHWMPVIKLSDVP DKNTGDPDMIELAKRVLALSRRA | 24/ 33.33% |
| 24 | MSQSNTPLKRKKTENGYSENGSTTGATSNQIRELKRATTVDYVYR DQESKDKIKPLNGFVTPLLTDLYQITMAYSLWKNNRHEIPAVFDLY FRKSPFGGEFTVFAGLEEVIRFVSDFHYTKEEVGFIKEMIPDCEQEFL DYLSKLDSSSVTLYAMKEGSVVFPRVPLLRVEGPMILCQLFETTLLC LVNFASLVATNAARHRLAVGKEKVMLEFGLRRAQGPDGAMSASR YSYLGGADGTSNVLAHCFFGIPIRGTHAHSFITNYSGPDELLDASIK DTNGNVHNLLEMSQKYRDELGYTATNLSELVAFVAYARTFPNGLV ALVDTYDTLASGVPNFICVALALHQLGYKAVGIRLDSGDLSYLSKA SRKLFKQIGEQFKIDYFEKFSIVASNDLNEPTIIALNRQGHEIDVFAIG TNLVTCQAQPALGCVYKLVEINGSPRIKLSQEANKITLPGRKTAYRL FGSEGHPLVDLLVDDNDMIKDGSKQIPQVGKKVLCLHPFEEQKRVI VTPVQIEKLHHIVFEKGQLTMPLPALNDIREYCFQQISKVREDHLRN SNPTPYKVSVTKELFDTLHNLWLDSVPIKEMK | 25/ 24.08% |
| 25 | MEKKENGLDGKQSGRVINGPTNPMVTPLLNDLYQFTMAYAYWKA GKQSERSVFDLYFRKNPFGGEYTIFAGLEECIKFLANFNLTDEEIDFV RDSLPGCEEAFCDYLRGLDCSDIEVYAISEGSVVFPKVPLLRIEGPV AVVQLLETPFLNLINYASLVATNAARHRFVAGKSKLLLEFGARRAQ GPDGAISASKYCYLGGFDATSNVAAGKLFGIPLRGTHSHAFVSSFM SLDEIVDKVLRSSDGKSTCKDFICLVQTCLTKIQNSSSLQGIFSETNQ SELAAFISYALAFPNSFLALVDTYDVMKSGIPNFCAVALALNELGY KAVGIRLDSGDLAYLSTEVRKFFCAIERDLKVPDFGKMIVTASNDL NEETVDALNKQGHEVDAFGIGTNLVTCYAQAALGCVFKLVEINNQ PRIKLSEDVTKVSIPCKKRTYRLFGKEGYPLVDIMTGENEPPPKVGE RLLCRHPFNESKRAYVVPQRVEELLKCYWRGNADEAREELEPLKE LRNRCIKQLENMRPDHMRRLNPTPYKVSVSAKLYDFIHFLWLNEAP VGELH | 26/ 23.64% |
| 26 | MAYAYWKSGKMEDKAVFDLFFRKNPFKGEFAIFAGLSECLKFLSN FRFSDSDINYLKQILPHVDPQFWEYLSGLDASQVTMSAIPEGYVVFP KVPLIRLEGPLPVIQLLETTLLNLVNYASLVATNAARFRITAGESVQ LLEFGLRRAQGPDGGLSASKYCYIGGFDGTSNVLAGKLFGIPVRGT HAHAFVNSFSSLDEIKGNVELTKSYLNKISPILNVLENETNEGELTAF LSYAFAFPTSCLCLLDTYSVIKSGLPNFLAVALALNDYGYRVIGVRL DSGDLAYLSRKCREGFNLIADKFGLPWIKQLIIVASNDINEDTLRSL KEQLVEVNGEPRMKLSEDVEKINLPGKKNVFRLYSEDGLAILDLIQ LCTEKPPAVGKKILVRHPFNESKRAYVCPHKVELLYTDYWGDNCL MQSFSTLQETKQIASDSLKTLRQDIKRELNPTPYKVSVTNEMYHFL HDLWLKHAPIGELS | 27/ 23.28% |
| 27 | MAYAYWKSGKMEDKAVFDLFFRKNPFKGEFAIFAGLSECLKFLSN 2 FRFSDSDINYLKQILPHVDPQFWEYLSGLDASQVTMSAIPEGYVVFP KVPLIRLEGPLPVIQLLETTLLNLVNYASLVATNAARFRITAGESVQ LLEFGLRRAQGPDGGLSASKYCYIGGFDGTSNVLAGKLFGIPVRGT HAHAFVNSFSSLDEIKGNVELTKSYLNKISPILNVLENETNEGELTAF LSYAFAFPTSCLCLLDTYSVIKSGLPNFLAVALALNDYGYRVIGVRL DSGDLAYLSRKCREGFNLIADKFGLPWIKQLIIVASNDINEDTLRSL KEQLVEVNGEPRMKLSEDVEKINLPGKKNVFRLYSEDGLAILDLIQ | 28/ 23.23% |
| | LCTEKPPAVGKKILVRHPFNESKRAYVCPHKVELLYTDYWGDNCL MQSFSTLQETKQIASDSLKTLRQDIKRELNPTPYKVSVTNEMYHFL HDLWLKHAPIGELS | |
| 28 | NSVVQPLLTDLYQVTMAYAYWKTGKMEDKAVFDLFFRKNPFRGE FAIFAGLGECVKFLNNFRFSCSDIAYLQEILPNVDQGFWEYLSQLDA SQVVTMSAIPEGYVVFPKVPLIRLEGPLPVIQLLETTFLNLINYASLV ATNAARFRITAGDGVQLLEFGLRRAQGPDGGLSASKYCYIGGFDGT SNVLAGKLFGIPVRGTHAHAFVNSFSSLDDELQVNPEAVLPQQPKL SFCQLAMSWLTKISPILNILEHETNKGELKAFLAYAYAFPRTCLCLL DTYSVIKLNLNICIFPFMDIKLGYPIIYTLDTQGIVVILHSLIYFTGLVT NCWNTQGTTFRVECCNGRNNSSLNFYTLMCYCFPHRSGLPNFLAV ALALNEYGYRAIGVRLDSGDLAYLSRKCREAFVLIADQFESPWMK KFTVVASNDINEETLISLKEQKHEINSFGIGTHLVTCQKQPALGCVF KLVEVNGEPRMKLSEDVGKINLPGKKSVFRLYSEDGLAILDLIQLCS EKPPEVGKKILVRHPFIESKRAYVCPYKVELLYRDYWGNNGLTQSF PSLQELKKTASDSLKTLRQDIKRELNPTPYKVSVTNEMYNFLHELW LKYAPIGELS | 29/ 23.11% |
| 29 | MNDRELGCAGGQFMDRGRMNQNGVVQPLLTDLYQITMAYAYWK SDKTDDTAVFDLFFRNNPFHGEFTIFAGLEECLKFLDSFHYSQSDIE YLKQTLPEGIEHEFFEYLGNLTARDVTLYAIDEGTVAFPRVPIIKIEG PLIIVQLLETTLLTLVNYASLMATNAARYRMVAGKHVKLLEFGLRR AQGPDGGLSASKYSYTGGFDGTSNVLAGKLFNIPVKGTHAHAYITS FSSIGELKTRLIKHKQTGILEDLLEHAVRHRALLSHLLDVSTEESSEG ELAAMVSYAIAFPDGFMALVDTYDVKSRETEQANTEPKHINDIKSE NSPPKPKASPSTNGHQNGQGVGHVNGHKTTNGHQNGSAQNNGSR TPKDTDIQDTSTSTTTTKTTATATGTLKTTTTVSNGTAYLPKYVEKS FRSGLLNFSAVALALNDLGYHALGIRIDSGDLAYLSCLARETFEKV AERFKVPWFNKLTIVASNDINEDTILSLNEQGHKIDCFGIGTHLVTC QRQPALGCVYKLVEINGQPRIKLSQDVEKVTMPGNKNAYRLYSAD GHALIDLLQKVSEPPPAVGQKVLCRHPFQESKRAYVIPSHVESLYK VYWKSGKICQQLPTLEQVREKVQISLKTLRNDHKRTLNPTPYKVA VSDNLYNFIHDLWLQNAPIGELS | 30/ 22.73% |
| 30 | MNDRELGCAGGQFMDRGRMNQNGVVQPLLTDLYQITMAYAYWK SDKTDDTAVFDLFFRNNPFHGEFTIFAGLEECLKFLDSFHYSQSDIE YLKQTLPEGIEHEFFEYLGNLTARDVTLYAIDEGTVAFPRVPIIKIEG PLIIVQLLETTLLTLVNYASLMATNAARYRMVAGKHVKLLEFGLRR AQGPDGGLSASKYSYTGGFDGTSNVLAGKLFNIPVKGTHAHAYITS FSSIGELKTRLIKHKQTGILEDLLEHAVRHRALLSHLLDVSTEESSEG ELAAMVSYAIAFPDGFMALVDTYDVKRSGLLNFSAVALALNDLGY HALGIRIDSGDLAYLSCLARETFEKVAERFKVPWFNKLTIVASNDIN EDTILSLNEQGHKIDCFGIGTHLVTCQRQPALGCVYKLVEINGQPRI KLSQDVEKVTMPGNKNAYRLYSADGHALIDLLQKVSEPPPAVGQK VLCRHPFQESKRAYVIPSHVESLYKVYWKSGKICQQLPTLEQVREK VQISLKTLRNDHKRTLNPTPYKVAVSDNLYNFIHDLWLQNAPIGEL S | 31/ 22.35% |
| 31 | MLEYGFKDDSLSLHTDLYQINMAETYWRDGIHEKKAIFELFFRRLP FENGYAVFAGLEKAIEYLENFKFTDSDLSYLQDELGYHEDFIEYLR GLSFTGSLYSMKEGELVFNNEPIMRVEAPLVEAQLIETALLNIVNYQ TLIATKAARIKGVIGDEVALEFGTRRAHEMDAAMWGARAALIGGF SATSNVRAGKRFNIPVSGTHAHALVQAYRDEYTAFKKYAETHKDC VFLVDTYDTLRSGMPNAIRVAKEFGDRINFIGIRLDSGDLAYLSKKA RKMLDEAGFTDAKVIASSDLDEHTIMNLKAQGARIDVWGVGTKLI TAYDQPALGAVYKLVAIEEDGKMVDTIKISSNPEKVTTPGRKKVYR IINQSNHHSEGDYIALYDEQVNDQKRLRMFHPVHTFISKFVTNFYA | 32/ 22.12% |
| | KDLHELIFEKGILCYQNPEISDIQQYVQDNLSLLWEEYKRISKPEEYP VDLSEDCWSNKMQRIHEVKSRIEEELEEE | |
| 32 | MSFILPSESIDGTHPCNGLHMNGQDSLVQPLLTDFYQITMCYAYWK TGTHNEPAVFDVFFRKNPFQGEFTVFAGLEDCLRFVENFKFSQSDID YVKKILPENAEPEFYEYLETLNGSHLTIEAVAEGSVVFPKVPLLTIN GPLAMCQLIETSILNLVNYASLVATNAARFRQASGWKIQLLEFGLR RAQGPNGGLTASKYCYIGGFDATSNVLAGKLYGIPVKGTQAHSFIC SFSSPAELKVRLLNHKITNEKADLFQISMEKRAWLLDQFSWKAALS EVSDGELSAFVAYAIAFPDTFLALIDTYDVIRSGVVNFVAVSLALHD LGYRSMGCRIDSGDLSYLSKELRECFVKVSTLKGEYKFFEKMSIVA SNDINEETIMSLNDQQHEINAFGVGTHLVTCQKQPALGCVYKLVAQ SAQPKIKLSQDVTKITIPGKKKCYRIFGKNGYAILDLMMLEDEPEPQ PNQQILCRHPFEESKRALVNANKIIKLHNVYWKDGEMITPLPTLNEI KEHVNESIRSTLRQDHRRYLNPTPYKVSVSERLYQFLHTLWLQNAP IGQLE | 33/ 20.71% |
| 33 | MAIRQHVGALFTDLYEVTMAQAYWAERMSGTAVFEIFFRKLPPGR SYIMAAGLADVVEFLEAFRFDEQDLRYLRGLGQFSDEFLRWLAGV RFTGDVWAAPEGTVIFPNEPAVQLIAPIIEAQLVETFVLNQIHLQSVL ASKAARVVAAARGRPVVDFGARRAHGTDAACKVARTSYLAGAA GTSNLLAARQYGIPTFGTMAHSFVQAFDSEVAAFEAFARLYPATML LVDTYDTLRGVDHVIELAKRLGNRFDVRAVRLDSGDLDELSKATR ARLDTAGLEQVEIFASSGLDENRIAALLAARCPIDGFGVGTQLVVA QDAPALDMAYKLVAYDGSGRTKFSSGKVIYPGRKQVFRKLEHGVF CGDTLGEHGENLPGDPLLVPIMTNGRRIRQHAPTLDGARDWARQQI DALPPELRSLEDTGYSYPVAVSDRIVGELARLRHADTAEAHPGSNV VGAKAKRP | 34/ 19.91% |
| 34 | MGPPPAARRREGEPDNQDPAGLLTDKYELTMLAAALRDGSANRPT 3 TFEVFARRLPTGRRYGVVAGTGRLLEALPQFRFDADACELLAQFLD PATVRYLREFRFRGDIDGYAEGELYFPGSPVLSVRGSFAECVLLETL VLSIFNHDTAIASAAARMVSAAGGRPLIEMGSRRTHERAAVAAAR AAYIAGFAASSNLAAQRRYGVPAHGTAAHAFTMLHAQHGGPTEL AERAAFRAQVEALGPGTTLLVDTYDVTTGVANAVAAAGAELGAIR IDSGELGVLARQAREQLDRLGATRTRIVVSGDLDEFSIAALRGEPVD SYGVGTSLVTGSGAPTANMVYKLVEVDGVPVQKRSSYKESPGGRK EALRRSRATGTITEELVHPAGRPPVIVEPHRVLTLPLVRAGQPVADT SLAAARQLVASGLRSLPGDGLKLAPGEPAIPTRTIPA | 35/ 19.79% |

| | | |
|---|---|---|
| * Underline: 212-234 motif * Sequence homology: based on the amino acid sequence of *Saccharomyces cerevisiae* | | |

According to one embodiment of the present invention, the nicotinate phosphoribosyltransferase (NPT) may be encoded by the nucleotide sequence of SEQ ID NO: 1, and may consist of at least one of the amino acid sequences of SEQ ID NOs: 2 to 35.

The nucleotide sequence or amino acid sequence of the nicotinate phosphoribosyltransferase (NPT) according to the present invention may consist of, or essentially include, a sequence having at least 70%, 75%, 80%, 85%, 90%, 95%, 96%, 97%, 98%, 99%, or 100% homology or identity to the nucleotide sequence of SEQ ID NO: 1 or to any of the amino acid sequences of SEQ ID NOs: 2 to 35, and may retain its original function. Herein, "homology" or "identity" refers to the percent identity between two sequences when a reference nucleotide or amino acid sequence and any other nucleotide or amino acid sequence are aligned to correspond to each other as much as possible.

As used herein, "enhanced activity" or "enhancement of activity" means that the activity of a target polypeptide or protein is increased compared to its endogenous activity. Such enhancement of activity includes: a case in which the activity of the polypeptide itself is increased compared to the activity of the polypeptide possessed by the original microorganism, i.e., the wild-type or pre-modification microorganism, due to a nucleotide modification of the gene encoding the polypeptide; a case in which the copy number of the gene encoding the polypeptide is increased; a case in which the overall level of polypeptide activity (expression level) is higher than that of the original microorganism due to increased expression or increased translation of the target gene resulting from a modification of the regulatory region of the gene encoding the polypeptide; a case in which a foreign gene encoding the polypeptide is introduced; and combinations thereof.

Herein, the nucleotide modification refers to a difference from the original polynucleotide sequence resulting from deletion, substitution, addition, or a combination thereof of all or part of the polynucleotide sequence of the gene encoding the polypeptide. The regulatory region modification refers to a difference from the original polynucleotide sequence resulting from deletion, substitution, addition, or a combination thereof of all or part of the polynucleotide sequence of elements constituting a regulatory region (regulatory sequence) such as a promoter, an enhancer, or a transcription terminator, and may, for example, involve replacement with a strong promoter so as to increase gene expression. Herein, deletion refers to a modification in which a base, nucleotide, polynucleotide, or nucleic acid is removed; substitution refers to a modification in which a base, nucleotide, polynucleotide, or nucleic acid is changed to another base, nucleotide, polynucleotide, or nucleic acid; and addition refers to a modification in which another base, nucleotide, polynucleotide, or nucleic acid is added.

The increase in copy number means that one or more copies of the polynucleotide sequence of the gene encoding the polypeptide are introduced into the chromosome of the original microorganism. The introduction of a foreign gene means that a polynucleotide sequence of a foreign gene encoding a polypeptide having the same or similar activity as the polypeptide is introduced; the foreign gene may encode a polypeptide that is present or absent in the original microorganism, and the introduction of the foreign gene may result in expression of the polypeptide, thereby increasing its activity.

According to one embodiment of the present invention, the enhancement of the activity of nicotinate phosphoribosyltransferase (NPT) may be achieved by a nucleotide modification of the gene encoding nicotinate phosphoribosyltransferase, an increase in the copy number of the gene, a modification of the regulatory region of the gene, introduction of a foreign gene, or a combination thereof.

According to one embodiment of the present invention, the nicotinate phosphoribosyltransferase (NPT) or the gene encoding it may be derived from yeast, and may be, for example, but not limited to, the nicotinate phosphoribosyltransferase (NPT) endogenous to *Saccharomyces cerevisiae*.

The mutant microorganism according to the present invention may further have an attenuated activity of nicotinamidase.

As used herein, "nicotinamidase" is an enzyme that converts nicotinamide (NAM) into nicotinic acid (NA); by controlling the activity of nicotinamidase, production of nicotinamide mononucleotide (NMN) from nicotinamide (NAM) can be induced.

The nicotinamidase of the present invention may be, but is not limited to, a polypeptide encoded by the *PNC1* gene that has nicotinamidase activity.

As used herein, "attenuated activity" or "activity attenuation" means that the activity of a target polypeptide or protein is decreased compared to its endogenous activity, or that the activity is absent, and may be used interchangeably with terms such as inactivation, deficiency, reduction, or decrease. Such activity attenuation may include, but is not limited to: a case in which the activity of the polypeptide itself is decreased or eliminated compared to the activity of the polypeptide possessed by the original microorganism, i.e., the wild-type or pre-modification microorganism, due to a nucleotide modification of the gene encoding the polypeptide; a case in which the overall level of polypeptide activity (expression level) is lower than that of the original microorganism due to inhibited expression or inhibited translation of the target gene resulting from a modification of the regulatory region of the gene encoding the polypeptide; a case in which the polypeptide has no activity even though the gene encoding it is expressed; and combinations thereof.

Herein, the nucleotide modification refers to a difference from the original polynucleotide sequence resulting from deletion, substitution, addition, or a combination thereof of all or part of the polynucleotide sequence of the gene encoding the polypeptide. The regulatory region modification refers to a difference from the original polynucleotide sequence resulting from deletion, substitution, addition, or a combination thereof of all or part of the polynucleotide sequence of elements constituting a regulatory region such as a promoter or an enhancer, and may, for example, involve replacement with a weak promoter so as to decrease or suppress gene expression.

According to one embodiment of the present invention, the attenuation of nicotinamidase activity may be achieved by a nucleotide modification of the gene encoding nicotinamidase, a regulatory region modification, or a combination thereof.

According to one embodiment of the present invention, the nicotinamidase or the gene encoding it may be derived from yeast, and may be, for example, but not limited to, the nicotinamidase endogenous to *Saccharomyces cerevisiae*.

According to one embodiment of the present invention, the nicotinamidase may consist of the amino acid sequence of SEQ ID NO: 48, and the gene encoding the nicotinamidase may comprise the nucleotide sequence of SEQ ID NO: 49.

The amino acid sequence or nucleotide sequence of the nicotinamidase according to the present invention may consist of, or essentially include, a sequence having at least 70%, at least 80%, at least 90%, at least 98%, at least 99%, or at least 99.9% homology to the amino acid sequence of SEQ ID NO: 48 or the nucleotide sequence of SEQ ID NO: 49, and must retain its original function.

Furthermore, the mutant microorganism of the present invention may further have an attenuated activity of nicotinamide mononucleotide adenylyltransferase.

As used herein, "nicotinamide mononucleotide adenylyltransferase (NMNAT)" is an enzyme that produces NAD⁺ using nicotinamide mononucleotide (NMN) as a substrate; by controlling the activity of nicotinamide mononucleotide adenylyltransferase (NMNAT), the amount of NMN produced can be increased.

The nicotinamide mononucleotide adenylyltransferase (NMNAT) of the present invention may be, but is not limited to, a polypeptide encoded by the *NMA1, NMA2*, or *POF1* gene that has nicotinamide mononucleotide adenylyltransferase activity.

According to one embodiment of the present invention, the attenuation of the activity of nicotinamide mononucleotide adenylyltransferase (NMNAT) may be achieved by a nucleotide modification of the gene encoding nicotinamide mononucleotide adenylyltransferase, a regulatory region modification, or a combination thereof.

According to one embodiment of the present invention, the nicotinamide mononucleotide adenylyltransferase (NMNAT) or the gene encoding it may be derived from yeast, and may be, for example, but not limited to, the nicotinamide mononucleotide adenylyltransferase (NMNAT) endogenous to *Saccharomyces cerevisiae*.

According to one embodiment of the present invention, the nicotinamide mononucleotide adenylyltransferase (NMNAT) may be encoded by the *NMA1* gene (SEQ ID NO: 43) and consist of the amino acid sequence of SEQ ID NO: 42; may be encoded by the *NMA2* gene (SEQ ID NO: 45) and consist of the amino acid sequence of SEQ ID NO: 44; or may be encoded by the POF1 gene (SEQ ID NO: 47) and consist of the amino acid sequence of SEQ ID NO: 46.

The amino acid sequence or nucleotide sequence of the nicotinamide mononucleotide adenylyltransferase (NMNAT) according to the present invention may consist of, or essentially include, a sequence having at least 70%, at least 80%, at least 90%, at least 98%, at least 99%, or at least 99.9% homology to the amino acid sequence of SEQ ID NO: 42, 44, or 46, or the nucleotide sequence of SEQ ID NO: 43, 45, or 47, and must retain its original function.

Furthermore, the mutant microorganism of the present invention may further have an enhanced activity of nicotinamide phosphoribosyltransferase.

As used herein, "nicotinamide phosphoribosyltransferase (NAMPT)" is an enzyme present in some bacteria and mammals that converts nicotinamide (NAM) into nicotinamide mononucleotide (NMN); the yeast-derived (e.g., *Saccharomyces cerevisiae*) nicotinamide phosphoribosyltransferase (NAMPT) has low substrate activity toward nicotinamide (NAM), which limits the mass production of nicotinamide mononucleotide (NMN) from nicotinamide (NAM) in yeast. In the present invention, the production yield of nicotinamide mononucleotide (NMN) can be increased by introducing nicotinamide phosphoribosyltransferase (NAMPT) derived from microorganisms other than yeast that are known to have high NAMPT activity, namely *Chitinophaga pinensis* and *Haemophilus ducreyi* (Shoji, et al., Metabolic Engineering. 2021 May 65:167-177.; Sorci, L. et al., Journal of Biological Chemistry. 2010 Dec 10;285(50):39490-9.).

The nicotinamide phosphoribosyltransferase (NAMPT) of the present invention may be, but is not limited to, a polypeptide encoded by the *NAMPT* gene that has nicotinamide phosphoribosyltransferase activity.

According to one embodiment of the present invention, the enhancement of the activity of nicotinamide phosphoribosyltransferase (NAMPT) may be achieved by introducing a gene encoding nicotinamide phosphoribosyltransferase (NAMPT) derived from *Chitinophaga pinensis* or *Haemophilus ducreyi*.

According to one embodiment of the present invention, the *Chitinophaga pinensis*-derived nicotinamide phosphoribosyltransferase (NAMPT) may consist of the amino acid sequence of SEQ ID NO: 38, and the gene encoding the nicotinamide phosphoribosyltransferase (NAMPT) may comprise the nucleotide sequence of SEQ ID NO: 39.

According to one embodiment of the present invention, the *Haemophilus ducreyi*-derived nicotinamide phosphoribosyltransferase (NAMPT) may consist of the amino acid sequence of SEQ ID NO: 40, and the gene encoding the nicotinamide phosphoribosyltransferase (NAMPT) may comprise the nucleotide sequence of SEQ ID NO: 41.

The amino acid sequence or nucleotide sequence of the nicotinamide phosphoribosyltransferase (NAMPT) according to the present invention may consist of, or essentially include, a sequence having at least 70%, at least 80%, at least 90%, at least 98%, at least 99%, or at least 99.9% homology to the amino acid sequence of SEQ ID NO: 38 or 40, or the nucleotide sequence of SEQ ID NO: 39 or 41, and must retain its original function.

Furthermore, the mutant microorganism of the present invention may further have an enhanced activity of phosphoribosylpyrophosphate synthase.

As used herein, "phosphoribosylpyrophosphate synthase (PRS)" is an enzyme that converts ribose 5-phosphate (ribose-5P) into phosphoribosyl pyrophosphate (PRPP), thereby supplying phosphoribosyl pyrophosphate (PRPP) required for the NMN production pathway.

The phosphoribosylpyrophosphate synthase (PRS) of the present invention may be, but is not limited to, a polypeptide encoded by the PRS1 gene that has phosphoribosylpyrophosphate synthase activity.

According to one embodiment of the present invention, the enhancement of the activity of phosphoribosylpyrophosphate synthase (PRS) may be achieved by a nucleotide modification of the gene encoding phosphoribosylpyrophosphate synthase, an increase in the copy number of the gene, a modification of the regulatory region of the gene, introduction of a foreign gene, or a combination thereof.

According to one embodiment of the present invention, the phosphoribosylpyrophosphate synthase (PRS) or the gene encoding it may be derived from yeast, and may be, for example, but not limited to, the phosphoribosylpyrophosphate synthase (PRS) endogenous to *Saccharomyces cerevisiae*.

According to one embodiment of the present invention, the phosphoribosylpyrophosphate synthase (PRS) may consist of the amino acid sequence of SEQ ID NO: 36, and the gene encoding the phosphoribosylpyrophosphate synthase (PRS) may comprise the nucleotide sequence of SEQ ID NO: 37.

The amino acid sequence or nucleotide sequence of the phosphoribosylpyrophosphate synthase (PRS) according to the present invention may consist of, or essentially include, a sequence having at least 70%, at least 80%, at least 90%, at least 98%, at least 99%, or at least 99.9% homology to the amino acid sequence of SEQ ID NO: 36 or the nucleotide sequence of SEQ ID NO: 37, and must retain its original function.

As used herein, "having enhanced nicotinamide mononucleotide productivity" means that the productivity of nicotinamide mononucleotide (NMN) is increased compared to that of the strain before mutation (the parent strain). The parent strain refers to the wild-type or mutant microorganism that is the subject of mutation, and includes a microorganism that is itself directly mutated or a microorganism that is transformed with a recombinant vector or the like.

In the present invention, the parent strain refers to a microorganism before its traits are changed by a genetic mutation caused by natural or artificial factors; for example, it may be a microorganism that does not produce NMN because it lacks an enzyme acting in the nicotinamide mononucleotide (NMN) production pathway, or it may be an NMN-producing microorganism expressing an enzyme involved in the NMN production pathway or a gene encoding such an enzyme. For example, it may be a wild-type genus *Saccharomyces* or a genus *Saccharomyces* mutated therefrom.

The genus Saccharomyces may be one known in the art, and may be, for example, but is not limited to, *Saccharomyces arboricolus, Saccharomyces bayanus, Saccharomyces bulderi, Saccharomyces cariocanus, Saccharomyces cariocus, Saccharomyces cerevisiae, Saccharomyces chevalieri, Saccharomyces dairenensis, Saccharomyces ellipsoideus, Saccharomyces eubayanus, Saccharomyces exiguous, Saccharomyces florentinus, Saccharomyces fragilis, Saccharomyces kudriavzevii, Saccharomyces martiniae, Saccharomyces mikatae, Saccharomyces monacensis, Saccharomyces norbensis, Saccharomyces paradoxus, Saccharomyces pastorianus, Saccharomyces spencerorum, Saccharomyces turicensis, Saccharomyces unisporus, Saccharomyces uvarum*, and *Saccharomyces zonatus*.

According to one embodiment of the present invention, the mutant microorganism may be *Saccharomyces cerevisiae*.

The mutant microorganism having enhanced nicotinamide mononucleotide (NMN) productivity according to the present invention has enhanced activity of nicotinate phosphoribosyltransferase (NPT); the NMN productivity may be further enhanced by additionally including a case in which the activity of one or more of nicotinamidase and nicotinamide mononucleotide adenylyltransferase (NMNAT) is further attenuated, a case in which the activity of one or more of nicotinamide phosphoribosyltransferase (NAMPT) and phosphoribosylpyrophosphate synthase (PRS) is further enhanced, or a combination thereof.

Specifically, the mutant microorganism having enhanced nicotinamide mononucleotide (NMN) productivity exhibits an increased NMN-producing ability compared to the parent strain; in particular, the amount of NMN produced may be increased by at least 1%, 5%, 10%, 15%, 20%, 25%, 30%, 35%, 40%, 45%, 50%, 55%, 60%, 65%, 70%, 75%, 80%, 85%, 90%, 95%, or 100%, or may be increased by 1.1-fold, 1.5-fold, 2-fold, 2.5-fold, 3-fold, 3.5-fold, 4-fold, 4.5-fold, 5-fold, 5.5-fold, 6-fold, 6.5-fold, 7-fold, 7.5-fold, 8-fold, 8.5-fold, 9-fold, 9.5-fold, 10-fold, 20-fold, 30-fold, 40-fold, 50-fold, 60-fold, 70-fold, 80-fold, 90-fold, or 100-fold compared to the parent strain, but is not limited thereto. For example, the mutant microorganism having enhanced expression of nicotinamide mononucleotide (NMN) may have an NMN production amount increased by 0.5-fold or more, specifically by 0.5- to 20-fold (preferably 1- to 10-fold), compared to the parent strain.

A composition comprising the mutant microorganism according to the present invention may be used as a composition for producing nicotinamide mononucleotide (NMN).

The mutant microorganism according to one embodiment of the present invention may be implemented through a recombinant vector comprising a gene encoding nicotinate phosphoribosyltransferase (NPT) for introducing said gene into a parent strain.

Furthermore, the mutant microorganism may be implemented through a recombinant vector for deleting or removing a gene encoding one or more of nicotinamidase and nicotinamide mononucleotide adenylyltransferase (NMNAT), or for introducing a gene encoding one or more of nicotinamide phosphoribosyltransferase (NAMPT) and phosphoribosylpyrophosphate synthase (PRS).

As used herein, "vector" means any type of nucleic acid sequence-carrying construct used as a means for delivering and expressing a gene of interest in a host cell. Unless otherwise specified, the vector may mean that the carried nucleic acid sequence is inserted into and expressed within the host cell genome and/or expressed independently. Such a vector includes the essential regulatory sequences operably linked so that the inserted gene is expressed; "operably linked" means that a gene of interest and its regulatory sequence are functionally linked to each other in a manner that enables gene expression, and "regulatory sequence" includes a promoter sequence for carrying out transcription, any operator sequence for regulating transcription, a sequence encoding a suitable mRNA ribosome binding site, and sequences that regulate the termination of transcription and translation.

The vector used in the present invention is not particularly limited as long as it is capable of replicating in a host cell, and any vector known in the art may be used. Examples of the vector include natural or recombinant plasmids, cosmids, viruses, and bacteriophages. For example, phage vectors or cosmid vectors include pWE15, M13, λMBL3, λMBL4, λIXII, λASHII, λAPII, λt10, λt11, Charon4A, and Charon21A; plasmid vectors include the pBR series, pUC series, pBluescriptII series, pGEM series, pTZ series, pCL series, and pET series, but are not limited thereto.

The vector may typically be constructed as a vector for cloning or a vector for expression. The expression vector may be a conventional one used in the art for expressing a foreign gene or protein in plants, animals, or microorganisms, and may be constructed by various methods known in the art.

As used herein, "recombinant vector" may be constructed using a prokaryotic or eukaryotic cell as a host, and may be capable of replicating independently of the host cell's genome or may be integrated into the genome itself. The host cell, in which the vector is replicable, may include an origin of replication, which is a specific nucleotide sequence at which replication is initiated. For example, when the vector used is an expression vector and a prokaryotic cell is used as the host, it generally includes a strong promoter capable of driving transcription (e.g., the pLλ promoter, the CMV promoter, the trp promoter, the lac promoter, the tac promoter, the T7 promoter), a ribosome binding site for initiation of translation, and transcription/translation termination sequences. When a eukaryotic cell is used as the host, an origin of replication that operates in the eukaryotic cell and is included in the vector may include, but is not limited to, the f1 origin of replication, the SV40 origin of replication, the pMB1 origin of replication, the adenoviral origin of replication, the AAV origin of replication, and the BBV origin of replication. In addition, a promoter derived from the genome of a mammalian cell (e.g., the metallothionein promoter) or a promoter derived from a mammalian virus (e.g., the adenovirus late promoter, the vaccinia virus 7.5K promoter, the SV40 promoter, the cytomegalovirus promoter, the HSV tk promoter) may be used, and it generally has a polyadenylation sequence as the transcription termination sequence.

The recombinant vector may include a selection marker, which is used to select a transformant (host cell) that has been transformed with the vector; because only the cells expressing the selection marker can survive in a medium treated with the corresponding selection agent, the selection of transformed cells is possible. Representative examples of the selection marker include, but are not limited to, ampicillin, kanamycin, streptomycin, and chloramphenicol.

A transformant can be made by inserting the recombinant vector into a host cell, and the transformant may be obtained by introducing the recombinant vector into a suitable host cell. The host cell may be any host cell known in the art that is capable of stably and continuously cloning or expressing the expression vector.

When transforming a prokaryotic cell to prepare a recombinant microorganism, the host cell may be, but is not limited to, Escherichia coli such as E. coli DH5α, E. coli JM109, E. coli BL21, E. coli RR1, E. coli LE392, E. coli B, E. coli X 1776, E. coli W3110, or E. coli XL1-Blue; the genus *Corynebacterium*; the genus *Bacillus* such as *Bacillus subtilis* or *Bacillus thuringiensis*; or various enterobacteria such as *Salmonella typhimurium, Serratia marcescens*, and the genus *Pseudomonas*.

When transforming a eukaryotic cell to prepare a recombinant microorganism, the host cell may be, but is not limited to, yeast (e.g., *Saccharomyces cerevisiae*), insect cells, plant cells, and animal cells, for example, Sp2/0, CHO K1, CHO DG44, PER.C6, W138, BHK, COS7, 293, HepG2, Huh7, 3T3, RIN, and MDCK cell lines.

As used herein, "transformation" means a phenomenon in which exogenous DNA is introduced into a host cell to artificially cause a genetic change, and "transformant" means a host cell into which exogenous DNA has been introduced and which stably maintains expression of a gene of interest.

In the transformation, an appropriate vector introduction technique is selected depending on the host cell so that the target gene or a recombinant vector comprising it can be expressed within the host cell. For example, the vector may be introduced by electroporation, heat-shock, calcium phosphate (CaPO₄) precipitation, calcium chloride (CaCl₂) precipitation, microinjection, the polyethylene glycol (PEG) method, the DEAE-dextran method, the cationic liposome method, the lithium acetate-DMSO method, or a combination thereof, but is not limited thereto. The transformed gene may be included without limitation, whether it is inserted into the chromosome of the host cell or located extrachromosomally, as long as it can be expressed within the host cell.

The transformant includes a cell transfected, transformed, or infected in vivo or in vitro with the recombinant vector according to the present invention, and may be used interchangeably with the terms recombinant host cell, recombinant cell, or recombinant microorganism.

The transformant of the present invention may exclude humans.

The genes inserted into the recombinant vector for transformation of the present invention may be substituted into a host cell such as yeast through homologous recombination crossover.

According to one embodiment of the present invention, the host cell may be yeast; for example, the yeast may be of the genus *Saccharomyces* or may be *Saccharomyces cerevisiae*.

In addition, another aspect of the present invention provides a method for producing nicotinamide mononucleotide (NMN), comprising steps of: culturing the mutant microorganism in a medium; and recovering nicotinamide mononucleotide (NMN) from the mutant microorganism or from the medium in which the mutant microorganism has been cultured.

The culturing may be carried out according to a suitable medium and culture conditions known in the art, and a person skilled in the art could readily adjust and use the medium and culture conditions. Specifically, the medium may be, but is not limited to, a liquid medium. The culturing method may include, but is not limited to, batch culture, continuous culture, fed-batch culture, or a combination thereof.

According to one embodiment of the present invention, the medium must meet the requirements of the specific strain in an appropriate manner, and may be appropriately modified by a person skilled in the art. The culture medium for yeast may refer to, but is not limited to, known literature (Folia Microbiol. 38(2), 141-146 (1993), etc.).

According to one embodiment of the present invention, the medium may contain various carbon sources, nitrogen sources, and trace element components. Carbon sources that can be used include sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture, but are not limited thereto. Nitrogen sources that can be used may include peptone, yeast extract, meat extract, malt extract, corn steep liquor, soybean flour, and urea, or inorganic compounds such as ammonium sulfate, ammonium chloride, ammonium phosphate, ammonium carbonate, and ammonium nitrate. The nitrogen sources may likewise be used individually or as a mixture, but are not limited thereto. Phosphorus sources that can be used may include, but are not limited to, potassium dihydrogen phosphate or dipotassium hydrogen phosphate, or corresponding sodium-containing salts. In addition, the culture medium may contain, but is not limited to, metal salts such as magnesium sulfate or iron sulfate that are necessary for growth. In addition, essential growth substances such as amino acids and vitamins may be included. Suitable precursors may also be used in the culture medium. The medium or individual components may be added to the culture in a batchwise or continuous manner in a suitable way during the culturing process, but are not limited thereto.

According to one embodiment of the present invention, during culturing, the pH of the culture may be adjusted by adding a compound such as ammonium hydroxide, potassium hydroxide, ammonia, phosphoric acid, or sulfuric acid to the microbial culture in an appropriate manner. In addition, foam formation may be suppressed during culturing by using an antifoaming agent such as a fatty acid polyglycol ester. Furthermore, in order to maintain an aerobic state of the culture, oxygen or an oxygen-containing gas (e.g., air) may be injected into the culture. The temperature of the culture may generally be 20 to 45°C, for example, 25 to 40°C. The culture period may continue until the useful substance is obtained in a desired production amount, and may be, for example, 10 to 160 hours.

According to one embodiment of the present invention, the step of recovering nicotinamide mononucleotide (NMN) from the cultured mutant microorganism or from the medium in which the mutant microorganism has been cultured may collect or recover the nicotinamide mononucleotide (NMN) produced from the medium using a suitable method known in the art depending on the culturing method. For example, methods such as centrifugation, filtration, extraction, spraying, drying, evaporation, precipitation, crystallization, electrophoresis, fractional dissolution (e.g., ammonium sulfate precipitation), and chromatography (e.g., ion exchange, affinity, hydrophobic, and size exclusion) may be used, but are not limited thereto.

According to one embodiment of the present invention, the step of recovering nicotinamide mononucleotide (NMN) may comprise removing biomass by low-speed centrifugation of the culture medium and separating the resulting supernatant by ion exchange chromatography.

According to one embodiment of the present invention, the step of recovering nicotinamide mononucleotide (NMN) may include a process of purifying nicotinamide mononucleotide (NMN).

### Advantageous Effects

The mutant microorganism according to the present invention can produce nicotinamide mononucleotide in high yield by enhancing, attenuating, or suppressing the expression of enzymes such as nicotinate phosphoribosyltransferase that are involved in the nicotinamide mononucleotide production pathway.

### Brief Description of Drawings

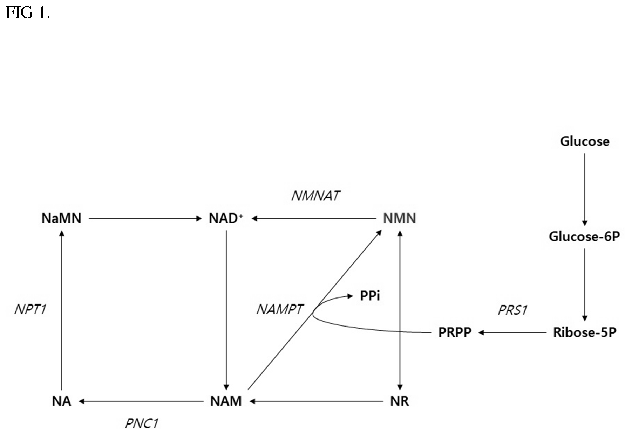

FIG. 1 is a diagram showing the substances and enzymes involved in a conventional NMN production pathway.
FIG. 2 is a diagram showing, according to one embodiment of the present invention, the process of introducing an NAMPT expression cassette comprising the foreign gene NAMPT while removing the endogenous gene *PNC1* in *Saccharomyces cerevisiae*.
FIG. 3 is a diagram showing, according to one embodiment of the present invention, the process of introducing the PDC promoter to overexpress the endogenous gene *PRS1* in *Saccharomyces cerevisiae*.
FIG. 4 is a graph showing the measured NMN production of the foreign-*NAMPT*-introduced and *PNC1*-removed mutant strains (RE01-1 and RE01-2) and the *PRS1*-overexpressing mutant strains (RE02-1 and RE02-2) according to one embodiment of the present invention.
FIG. 5 is a diagram showing, according to one embodiment of the present invention, the process of introducing the CCW12 promoter to overexpress the endogenous gene *NPT1* in *Saccharomyces cerevisiae*.
FIG. 6 is a graph showing the measured NMN production of the *NPT1-*overexpressing mutant strain (RE03) according to one embodiment of the present invention.
FIG. 7 is a diagram showing, according to one embodiment of the present invention, the process of introducing the kanamycin antibiotic resistance gene *KANMX* while removing *PNC1* in *Saccharomyces cerevisiae*.
FIG. 8 is a diagram showing, according to one embodiment of the present invention, the process of introducing an *NPT1* expression cassette comprising *NPT1* in *Saccharomyces cerevisiae*.
FIG. 9 is a graph showing the measured NMN production of the *NPT1*-promoter-enhanced mutant strain (YJ02) and the *NPT1*-2-copy-expressing mutant strain (YJ03) according to one embodiment of the present invention.
FIG. 10 is a diagram showing, according to one embodiment of the present invention, the process of introducing an *NPT1* expression cassette comprising *NPT1* while removing *PNC1* in *Saccharomyces cerevisiae*.
FIG. 11 is a graph showing the measured NMN production of the *PNC1-*removed mutant strain (DY2 pnc1::KanMX), the *NPT1*-2-copy-expressing mutant strain (DH01), and the *PNC1*-removed and *NPT1*-2-copy-expressing mutant strain (D452-2 pnc1::NPT1) according to one embodiment of the present invention.
FIG. 12 is a diagram showing, according to one embodiment of the present invention, the process of removing the NMNAT-related gene *POF1* in *Saccharomyces cerevisiae*.
FIG. 13 is a graph showing the measured NMN production of the *NPT1-*overexpressing mutant strain (YJ02), the *NPT1*-2-copy-expressing mutant strain (YJ03), and the *POF1*-removed mutant strain (YJ02 POF1Δ) according to one embodiment of the present invention.

### Detailed Description of Embodiments

Hereinafter, the present invention will be described in more detail. However, this description is presented by way of example only to aid understanding of the present invention, and the scope of the present invention is not limited by these illustrative descriptions.

### Example 1. Removal of Endogenous Gene PNC1 and Introduction of Foreign Gene NAMPT

### 1-1. Construction of PNC1-Removed and NAMPT-Introduced Mutant Strains

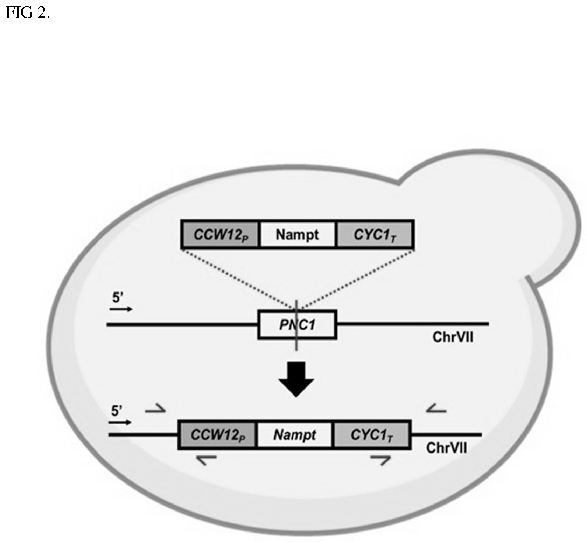

A mutant strain was constructed by removing *PNC*1, the endogenous gene of *Saccharomyces cerevisiae* involved in converting NAM to NA, and introducing *NAMPT* derived from *Chitinophaga pinensis* or *Haemophilus ducreyi* into the corresponding locus (see FIG. 2).

The CRISPR/Cas9 system was used to construct the mutant strains, and the plasmid expressing each gRNA nucleotide sequence was constructed by a Polymerase Chain Reaction (PCR)-based fast cloning method. The gRNA sequence was designed using the CHOPCHOP software (https://chopchop.cbu.uib.no/), and the plasmid pRS42H-*PNC1* was constructed by PCR amplification using primers Kim1703 and Kim1704. The PCR product was treated with the restriction enzyme Dpn1, transformed into *E. coli* TOP10 (Invitrogen), and selected on LBA solid medium (containing 5 g/L yeast extract, 10 g/L tryptone, 10 g/L NaCl, and 100 µg/mL ampicillin). The gRNA nucleotide sequence of the resulting plasmid was confirmed and selected by Sanger sequencing using a T3 promoter primer (5***'***-CAATTAACCCTCACTAAA-3***'***, SEQ ID NO: 50).

To construct the NAMPT expression cassette, the nucleotide sequence of NAMPT derived from *Chitinophaga pinensis* or *Haemophilus ducreyi* was codon-optimized and prepared, and the CCW12 promoter, which provides a high expression level, was used. The NAMPT expression cassette was amplified by PCR using primers Kim1716 and Kim1717 and Q5^{®} High-Fidelity DNA Polymerase (initial denaturation: 98°C for 30 s; 30 cycles of 98°C for 5 s, 66°C for 10 s, and 72°C for 55 s; final extension: 72°C for 2 min), and was introduced into strain DY2 by the LiAc transformation method. Strain DY2 is a strain based on *Saccharomyces cerevisiae* D452-2 into which the xylose metabolism genes *XYL1, XYL2*, and *XYL3* were introduced, and in which the *PHO13* gene encoding phosphatase and the *ALD6* gene encoding acetaldehyde dehydrogenase were deleted (Jeong, Deokyeol, et al. PLOS ONE. 2020 Jul 27;15(7):e0236294.). The selection of the target strain was confirmed by colony PCR using primers Kim549 and Kim742, which yielded bands of 1.67 kb and 1.74 kb, respectively, and the strains into which *NAMPT* derived from *Chitinophaga pinensis* and *Haemophilus ducreyi* were introduced were designated RE01-1 and RE01-2, respectively.

The genes *PNC1* and *NAMPT*, the CCW12 promoter, the primers, and the gRNA used are shown in Table 3 below.

**[Table 3]**

| Name | | **Sequence (5'-3')** | **SEQ ID NO.** |
|---|---|---|---|
| *PNC1* | | ATGAAGACTTTAATTGTTGTTGATATGCAAAATGATTTTATTT CACCTTTAGGTTCCTTGACTGTTCCAAAAGGTGAGGAATTAA TCAATCCTATCTCGGATTTGATGCAAGATGCTGATAGAGACT GGCACAGGATTGTGGTCACCAGAGATTGGCACCCTTCCAGAC ATATTTCGTTCGCAAAGAACCATAAAGATAAAGAACCCTATT CAACATACACCTACCACTCTCCAAGGCCAGGCGATGATTCCA CGCAAGAGGGTATTTTGTGGCCCGTACACTGTGTGAAAAACA CCTGGGGTAGTCAATTGGTTGACCAAATAATGGACCAAGTGG TCACTAAGCATATTAAGATTGTCGACAAGGGTTTCTTGACTG ACCGTGAATACTACTCCGCCTTCCACGACATCTGGAACTTCC ATAAGACCGACATGAACAAGTACTTAGAAAAGCATCATACA GACGAGGTTTACATTGTCGGTGTAGCTTTGGAGTATTGTGTC AAAGCCACCGCCATTTCCGCTGCAGAACTAGGTTATAAGACC ACTGTCCTGCTGGATTACACAAGACCCATCAGCGATGATCCC GAAGTCATCAATAAGGTTAAGGAAGAGTTGAAGGCCCACAA CATCAATGTCGTGGATAAATAA | 49 |
| *NAMPT (Chitinophag a pinensis)* | | ATGACGAAGGAGAACTTGATCTTGCTGGCAGACGCGTATAAA TATAGCCATCATAAGTTATATATCCCGGGCACTGAATACATC TACTCATACTTTGAGAGTAGGGGCGGCAAATTTAACGAAACG GTGTTCTATGGACTACAGTATTTCCTTATGGAATATTTGCAGG GTGCTGTAATCACAAAAGAAAAACTTGATGAAGCCGAGGCA ACTTTATTAGAAGTCTTTGGGAGAAATGACGTATTCGACAGA ACCCGTTTCGAGTACATCATAGAAAAGCACGGTGGTAGGCTA CCGGTGAGAATCAAAGCCGTCCCTGAGGGAACCGTGACGG GGTGCGTAACGTCTTAATGACGATTGAGAATACTGACCCGAA CTGCTACTGGGTTACGAATTTTTTAGAGACGCTGTTAATGCA AATTTGGTACCCCTGTACCGTCGCGACCCTAAGCAGAGAGAT CAAAAAGACGGTTAAGCAGTACTATAATGAGACTGCAAGTG AGGCTGCTTTCGCTGGCATCGACTTCGTCTTAAACGATTTTGG GTTCAGGGGAGCCAGCTCCGTGGAGAGCGCCGGCATCGGAG GGTCTGCACACCTTATCAACTTCTCCGGTAGCGATACGCTGA TAGGCAGTACATTTGCCAAGAGATACTATCAAGCACCTGTGG CACCTGGGCTAAGCATTCCGGCGACGGAGCATTCAATAGTCA CCATGCTAGGTGAAGAGGGAGAATTGGAAATTTTTCGTCACA TTTTGAATGCTTTCCCAACAGGTACAATAGCCTGCGTGTCCG ACTCTTATAATATTCTAAGGGCCTGCAGAGAATACTGGGGAA CTGAACTAAAGGAACAAATACTGTCACGTCAGGGAACGCTA GTTATTAGGCCCGACAGCGGAGATGCGATTCAGACATTACTT AAAGTTTTCGAGATCCTGATGGAGACGTTCGGTTACACTGTG AACGAGAAAGGGTATAAAGTCCTTCCCCCGCAAGTCAGAGTT ATACAAGGGGATGGCATCTCCTATTCTAGTATACCACCTATA TTTGAGGCACTGAAGCAAGCAGGAATATCTGCGGAAAATCTA GTCCTGGGGATGGGCGGGGCTCTATTGCAGAGAGTCAATAGG GACACCCAAGAATACGCTCTAAAGTGTTCTTTCGCCCAGGTC AACGGCAAAGCCATAAATGTGCAAAAGAATCCTCTAGAACTT GACGCCAACGGCAATACGCGTGTTAGTTTCAAAAAAAGCAA ATCAGGGAAGCAAAAACTTGTAGTCGAGAACGGATTTACA CAAGTTTGCCGGAAAACGAGGCACCGGCACTGGCCGACCAG | 39 |
| | | CTTGTTACCGTTTCGAAGATGGGGAAATTAAAAAGCATAC TCATTCGAACAGATCAGGAAAAATGCCACGATTTAA | |
| *NAMPT (Haemophilus ducreyi)* | | ATGGACAATCTACTAAACTATAGCTCAAGGGCGTCTGCTATA CCGAGTTTGTTGTGCGATTTCTATAAGACATCCCACCGTATCA TGTACCCGGAGTGCAGTCAAATAATCTATTCCACGTTCACGC CAAGATCCAACGAACAGGCTCCATATTTGACGCAAGTCGTCT CATTCGGTTTCCAGGCCTTTATCATCAAGTATTTAATTCATTA CTTTAACGACAATTTCTTTTCCCGTGACAAGCATGACGTAGTT ACCGAATACTCCGCTTTTATTGAAAAGACTTTGCAGTTAGAA GACACCGGGGAGCACATTGCAAAGTTGCACGAATTAGGCTA CCTACCGATCCGTATAAAGGCTATCCCGGAGGGTAAAACGGT GGCCATCAAAGTTCCGGTCATGACTATCGAAAACACACACTC AGATTTCTTTTGGCTTACTAACTACTTAGAAACTCTAATTAAC GTATCTCTTTGGCAACCTATGACTTCCGCTTCCATTGCCTTTG CTTATCGTACAGCTCTGATCAAATTCGCAAATGAGACATGCG ATAATCAAGAACATGTCCCTTTCCAGTCCCATGATTTTAGCAT GCGTGGTATGTCCTCACTTGAGTCTGCCGAGACATCAGGCGC AGGACATCTAACTAGCTTCTTAGGTACTGATACAATCCCTGC GCTATCATTCGTCGAGGCGTACTATGGTTCATCTTCTCTAATT GGAACGAGTATTCCGGCATCCGAACATTCAGTCATGTCTAGT CACGGTGTGGATGAATTGAGTACATTCAGATATCTTATGGCT AAGTTTCCACACAATATGCTATCCATAGTCTCTGACACAACT GACTTCTGGCACAACATTACAGTTAATCTTCCCCTACTAAAA CAGGAGATCATTGCGAGGCCTGAAAATGCCCGTCTGGTTATA AGGCCCGATTCAGGCAATTTCTTTGCTATAATCTGTGGGGAC CCGACAGCTGACACCGAGCATGAGCGTAAGGGGTTGATCGA GTGTTTATGGGATATCTTCGGAGGGACAGTCAACCAAAAAGG CTACAAAGTCATCAACCCCCATATCGGAGCGATTTACGGTGA CGGGGTTACATACGAGAAGATGTTCAAAATCTTAGAGGGATT GCAGGCGAAGGGCTTTGCCTCATCCAACATTGTCTTTGGAGT AGGGGCTCAGACATACCAGCGTAATACGAGAGATACACTGG GGTTCGCCCTAAAGGCTACTAGCATAACGATAAACGGAGAG GAAAAGTCTATTTTCAAGAATCCGAAAACGGACGATGGCTTT AAGAAGAGTCAAAAGGGGAGGGTCAAAGTGCTGAGCAGAGA CACATATGTCGACGGATTGACAAGTGCGGACGACTTCTCCGA CGACCTACTAGAACTACTGTTTGAGGATGGTAAGCTATTGCG TCAAACAGATTTCGATGAGATACGTCAGAATCTGCTAGTGAG CAGAACGACCCTTTGA | 41 |
| CCW12 promoter *(Saccharomy ces cerevisiae* S288C) | | CCACCCATGAACCACACGGTTAGTCCAAAAGGGGCAGTTCAG ATTCCAGATGCGGGAATTAGCTTGCTGCCACCCTCACCTCAC TAACGCTGCGGTGTGCGGATACTTCATGCTATTTATAGACGC GCGTGTCGGAATCAGCACGCGCAAGAACCAAATGGGAAAAT CGGAATGGGTCCAGAACTGCTTTGAGTGCTGGCTATTGGCGT CTGATTTCCGTTTTGGGAATCCTTTGCCGCGCGCCCCTCTCAA AACTCCGCACAAGTCCCAGAAAGCGGGAAAGAAATAAAACG CCACCAAAAAAAAAAAAATAAAAGCCAATCCTCGAAGCGTG GGTGGTAGGCCCTGGATTATCCCGTACAAGTATTTCTCAGGA GTAAAAAAACCGTTTGTTTTGGAATTCCCCATTTCGCGGCCA CCTACGCCGCTATCTTTGCAACAACTATCTGCGATAACTCAG CAAATTTTGCATATTCGTGTTGCAGTATTGCGATAATGGGAG TCTTACTTCCAACATAACGGCAGAAAGAAATGTGAGAAAATT TTGCATCCTTTGCCTCCGTTCAAGTATATAAAGTCGGCATGCT TGATAATCTTTCTTTCCATCCTACATTGTTCTAATTATTCTTAT TCTCCTTTATTCTTTCCTAACATACCAAGAAATTAATCTTCTG TCATTCGCTTAAACACTATATCAATA | 51 |
| Primer | Kim1716 | CTTCCATAAGACCGACATGAACAAGTACTTCCACCCATGAAC CACACGGTTAGTC | 52 |
| | Kim1717 | CAATACTCCAAAGCTACACCGACAATGTAAAGGCCGCAAATT AAAGCCTTC | 53 |
| | Kim 1703 | AAAGCATCATACAGACGGTTTTAGAGCTAGAAATAGCAAG | 54 |
| | Kim1704 | GTCTGTATGATGCTTTTCTGATCATTTATCTTTCACTGCG | 55 |
| | Kim549 | GCATCCTTTGCCTCCGTTC | 56 |
| | Kim742 | AATAGGGACCTAGACTTCAG | 57 |
| gRNA; pRS42H-*PNC1* | | AGAAAAGCATCATACAGACGAGG | 58 |

### Example 2. Overexpression of Endogenous Gene PRS1

### 2-1. Construction of PNC1-Removed, NAMPT-Introduced, and PRS1- Overexpressing Mutant Strains

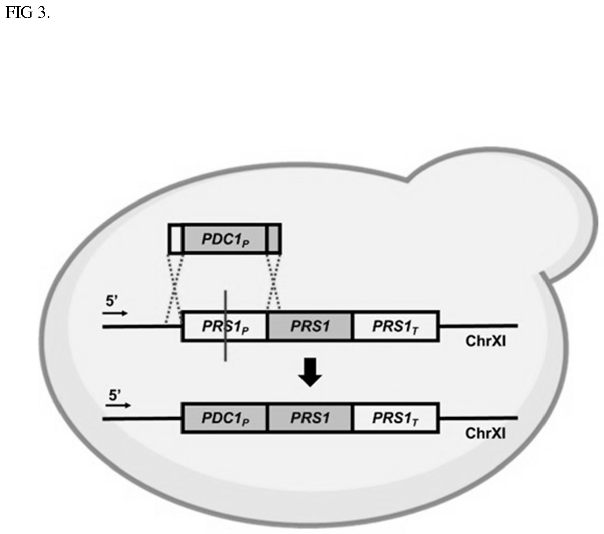

A mutant strain overexpressing the endogenous gene *PRS1* of *Saccharomyces cerevisiae* was constructed using the PDC1 promoter, which provides a high expression level (see FIG. 3).

The CRISPR/Cas9 system was used to construct the mutant strain, and the plasmid expressing each gRNA nucleotide sequence was constructed by a PCR-based fast cloning method. The gRNA sequence was designed using the CHOPCHOP software (https://chopchop.cbu.uib.no/), and the plasmid pRS42H-PRS1p was constructed by PCR amplification using primers Kim1638 and Kim1639. The PCR product was treated with the restriction enzyme Dpn1, transformed into *E. coli* TOP10 (Invitrogen), and selected on LBA solid medium (containing 5 g/L yeast extract, 10 g/L tryptone, 10 g/L NaCl, and 100 µg/mL ampicillin). The gRNA nucleotide sequence of the resulting plasmid was confirmed and selected by Sanger sequencing using a T3 promoter primer (5'-CAATTAACCCTCACTAAA-3', SEQ ID NO: 50).

The PDC1 promoter sequence (1.4 kb) to be inserted in place of the original promoter of *PRS1* was prepared by PCR using primers Kim1740 and Kim1737 and Q5^{®} High-Fidelity DNA Polymerase (initial denaturation: 98°C for 30 s; 30 cycles of 98°C for 5 s, 55°C for 10 s, and 72°C for 30 s; final extension: 72°C for 2 min). The plasmid pRS42H-PRS1p and the PDC1 promoter fragment were introduced into RE01-1 and RE01-2, which had been constructed in Example 1, by the LiAc transformation method. The selection of the target strain was confirmed by colony PCR using primers Kim1746 and Kim1657, which yielded a band of 1.4 kb, and the resulting strains were designated RE02-1 and RE02-2, respectively.

*PRS1*, the *PDC1* promoter, the primers, and the gRNA used are shown in Table 4 below.

**[Table 4]**

| Name | | **Sequence (5'-3')** | **SEQ ID NO.** |
|---|---|---|---|
| *PRS1* | | ATGCGTAAGTGTAAAATTTTGTTGGTAACTCTCACCCAG AGTTGGGTAATATGGTTTGCCAAAGATTAGGTATCGAAC CAGCTCCATGTACATTAAAAAAATTTGCAAATGGTGAAA CGTCAGTTCAAATCGGTGTATCTGTTCGTGACGAAGATGT GTATGTCATTCAATCTGGTTCTCCATCCATAAATGATGAC ATTATGGAACTGTTGATTTTAGTTTCTGCCTGTAGGGGCG GATCTGCTAGGAAAATTACAGCAGTCATTCCTCAATTTCC TTACTCTAAACAATGTAAAATGAAGAGACACAGAGGCGC TATTACTGCAAGGATGTTGGCCAATCTGTTGGTCATGGCT GGCGCTGATCATGTTGTGTCTATGGACCTTCATGCTTCTC AAATGCAGGGGTTTTCACCAAACCAGTCGATAACCTAT ACGGGGGCCCTAGTTTAGCGAAATGGATTAGAGAAAATG TTGAAGACTATGAAGATGCAGTAGTTGTCTCTAAGAATCC AGGTGGTACTAAAAGAGTTACAGCCCTGGCGGATTCTTT GAAGATCAACTTTGCTATGATCCATACTGATCGTCGTCGT TCTAAGGATCTGTATTCTCAAAACAAGGATTTGCAACAAC TAAAGTTGAGAAAGCAATCAATGTTGAGGAAAAATAGAC CGATCATCAGACAAGGTGACCATCCAAATGAGGAGGAAA ACATTATTTTAAGTAACGGTATTCAAACTGCTAGGATCCG TAACGGCCACGTTATCGGTGACGATGAGGCAGACGATGA TGAAGACGCTATCTTAGAATCTGACAGCGAATTGCATTCT ATTGATGGCTTAGACTCGCATGGCTTGGGCGGTACATATG ATGCTGTCGACTCCGAAGACGAAGAGGAAATCCCAGTTC TCTATAGGGAACAACTGATTACATTGGTCGGAAACGTCC GTGGTCGCTCTGCCATCATTCTAGATGATATGATTGATAG ACCTGGGTCATTTATTAGCGCTGCTGAACATTTGGTGCAA | 37 |
| | | AATTGTGGTGCCAAAAAGGTTTATGTTGTTGCTACTCACG GTATATTCACTGGAGACTGTTTGGAAGAACTTGAAAAGT CCGATGCCATTGACACAATTGTGGTCACAAATACATATCC GATTTCTGGCGAACGCATAGCTGGGTCCAAGAAATTGGT TACTATCGATGTTTCTCCAATTTTCGCAGAATGTATCCGT CGTGATCATTATGGTGAGAGTATTTCCGTTTTATTTGACT CTTTGGCAGCTCTGTAA | |
| PDC1 promoter (*Saccharomy ces cerevisiae* S288C) | | GATTTCATCATACTCCATACCTTCTTCCACCGTGTCAAGC AACTATGGGTAAAATCTTTATCTTCCGCTTTGCCATTTA AGGAGGAGGAAGATGTCAGAAGGAAAGAGGACGTCAAC TATGAAAGAAGATCAAGCGAGTCCATCGGCGACCTGCCG CACCGCGTAAACTCTCTTAACATACGGCCGTACAGAAAA AACGGCACTGGAGTGAGCAGTGTCTCCGACGATTTGGAT ATCGATTTCAACGATAGTTTTGCCTCAGAAGAAAGTTATA AACGTGCCGCTGCAGTGACATCTACCCTAAAGGCCAGAA TTGAAAAAATGAAGGCCAAATCAAGGCGGGAAGGGACA ACCAGGACGTAAAGGGTAGCCTCCCCATAACATAAACTC AATAAAATATATAGTCTTCAACTTGAAAAAGGAACAAGC TCATGCAAAGAGGTGGTACCCGCACGCCGAAATGCATGC AAGTAACCTATTCAAAGTAATATCTCATACATGTTTCATG AGGGTAACAACATGCGACTGGGTGAGCATATGTTCCGCT GATGTGATGTGCAAGATAAACAAGCAAGGCAGAAACTAA CTTCTTCTTCATGTAATAAACACACCCCGCGTTTATTTACC TATCTCTAAACTTCAACACCTTATATCATAACTAATATTT CTTGAGATAAGCACACTGCACCCATACCTTCCTTAAAAAC GTAGCTTCCAGTTTTGGTGGTTCCGGCTTCCTTCCCGATT CCGCCCGCTAAACGCATATTTTTGTTGCCTGGTGGCATTT GCAAAATGCATAACCTATGCATTTAAAAGATTATGTATGC TCTTCTGACTTTTCGTGTGATGAGGCTCGTGGAAAAAATG AATAATTTATGAATTTGAGAACAATTTTGTGTTGTTACGG TATTTTACTATGGAATAATCAATCAATTGAGGATTTTATG CAAATATCGTTTGAATATTTTCCGACCCTTTGAGTACTTT TCTTCATAATTGCATAATATTGTCCGCTGCCCCTTTTTCTG TTAGACGGTGTCTTGATCTACTTGCTATCGTTCAACACCA CCTTATTTTCTAACTATTTTTTTTTAGCTCATTTGAATCA GCTTATGGTGATGGCACATTTTTGCATAAACCTAGCTGTC CTCGTTGAACATAGGAAAAAAAAATATATAAACAAGGCT CTTTCACTCTCCTTGCAATCAGATTTGGGTTTGTTCCCTTT ATTTTCATATTTCTTGTCATATTCCTTTCTCAATTATTATTT TCTACTCATAACCTCACGCAAAATAACACAGTCAAATCA ATCAAA | 59 |
| primer | Kim1740 | CTTCAACTCTTTACTCGAAAAGTTGAAACAAGTGTCTATT GATTTCATCATACTCCATAC | 60 |
| | Kim1737 | GTGAGAGTTACCAACAAAATTTTACACTTACGCATTTTT TTTGATTGATTTGACTGTG | 61 |
| | Kim1638 | GTTATTGCAGAGATGTGGTTTTAGAGCTAGAAATAGCAA G | 62 |
| | Kim1639 | ACATCTCTGCAATAACCGAGATCATTTATCTTTCACTGCG | 63 |
| | Kim1746 | TATGTTCCGCTGATGTGATGTG | 64 |
| | Kim1657 | ACAGATCCTTAGAACGACGACG | 65 |
| gRNA; pRS42H-*PRS1*_{P} | | TCGGTTATTGCAGAGATGTGCGG | 66 |

### 2-2. Evaluation of NMN Productivity of PRS1-Overexpressing Mutant Strains

To evaluate the NMN productivity according to PRS1 overexpression, the NMN production amounts of the PRS1-overexpressing mutant strains RE02-1 and RE02-2 were compared with those of RE01-1 and RE01-2 from Example 1.

Each strain was inoculated into 10 mL of YPD medium (containing 10 g/L yeast extract, 20 g/L peptone, and 20 g/L glucose) and pre-cultured at 30°C and 250 rpm for 24 hours. The pre-culture was then inoculated at 0.5% (v/v) into fresh YPD medium and main-cultured at 30°C and 250 rpm for 11 hours. After the main culture, 50 mg of properly grown cells were disrupted using Y-PER^{™} yeast protein extraction reagent (Thermo Scientific) and protease inhibitor and used for evaluation of NMN productivity.

The cell lysate was reacted in a reaction buffer containing 20% (v/v) crude extract per 0.6 mL reaction mixture, 50 mM Tris-HCl (pH 7.5), 10 mM MgCl₂, 10 mM ATP, 40 mM NAM, and 5 mM PRPP. After a 12-hour reaction, the mixture was filtered through a 0.45 µm filter, and the NMN production amount was analyzed using high-performance liquid chromatography (HPLC) (Agilent Technologies 1260 Infinity, Agilent Technologies) equipped with a column (Supelcosil^{™} LC-18) and a UV detector (260 nm).

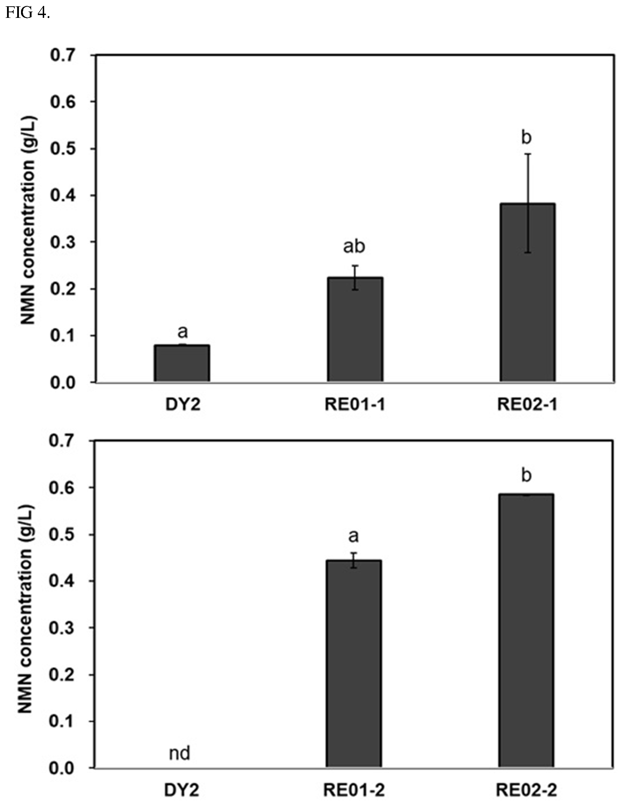

As a result, referring to FIG. 4, the overexpression of PRS1 significantly increased NMN production; in particular, the strain into which *Haemophilus ducreyi-*derived *NAMPT* was introduced (RE02-2) showed increased NMN production compared to the strain into which *Chitinophaga pinensis*-derived *NAMPT* was introduced (RE02-1).

### Example 3. Overexpression of Endogenous Gene NPT1

### 3-1. Construction of PNC1-Removed, NAMPT-Introduced, and NPT1-Overexpressing Mutant Strains

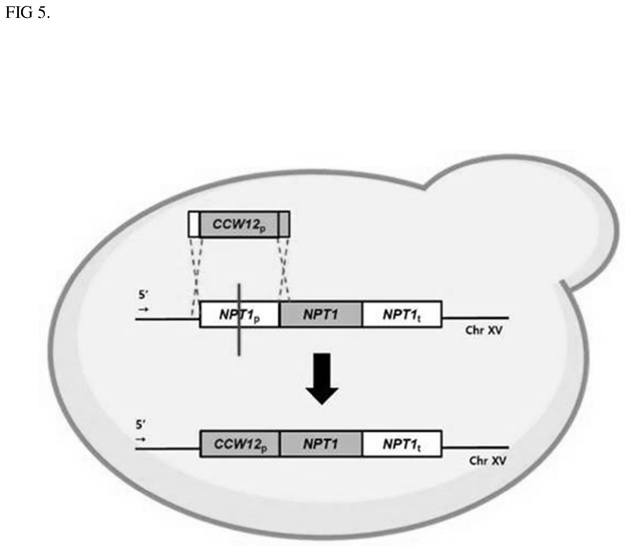

A mutant strain was constructed that overexpresses NPT1, the endogenous gene of *Saccharomyces cerevisiae* expected to have high structural similarity to NAMPT (see FIG. 5).

The CRISPR/Cas9 system was used to construct the mutant strain, and the plasmid expressing each gRNA nucleotide sequence was constructed by a PCR-based fast cloning method. The gRNA sequence was designed using the CHOPCHOP software (https://chopchop.cbu.uib.no/), and the plasmid pRS42H-NPT1p was constructed by PCR amplification using primers Kim1806 and Kim1807. The PCR product was treated with the restriction enzyme Dpn1, transformed into E. coli TOP10 (Invitrogen), and selected on LBA solid medium (containing 5 g/L yeast extract, 10 g/L tryptone, 10 g/L NaCl, and 100 µg/mL ampicillin). The gRNA nucleotide sequence of the resulting plasmid was confirmed and selected by Sanger sequencing using a T3 promoter primer (5'-CAATTAACCCTCACTAAA-3', SEQ ID NO: 50).

The *CCW12* promoter sequence to be inserted in place of the original promoter of *NPT1* was amplified by PCR using primers Kim1808 and Kim1809 and Q5^{®} High-Fidelity DNA Polymerase (initial denaturation: 98°C for 30 s; 30 cycles of 98°C for 5 s, 59°C for 10 s, and 72°C for 20 s; final extension: 72°C for 2 min), and was introduced into RE02-1, which had been constructed in Example 2-1, by the LiAc transformation method. The selection of the target strain was confirmed by colony PCR using primers Kim549 and Kim1811, which yielded a band of 0.65 kb, and the resulting strain was designated RE03.

*NPT1*, the primers, and the gRNA used are shown in Table 5 below.

**[Table 5]**

| Name | | **Sequence (5'-3')** | **SEQ ID NO.** |
|---|---|---|---|
| *NPT1* | | ATGTCAGAACCAGTGATAAAGTCTCTTTTGGACACAGACAT GTACAAGATTACGATGCATGCTGCTGTCTTCACTAATTTTC CAGATGTTACAGTTACTTATAAATATACCAACAGGTCGTCC CAATTGACCTTCAATAAGGAAGCCATTAATTGGTTGAAAG AGCAATTTTCGTATTTGGGAAATTTGAGGTTCACAGAAGAG GAAATTGAATACTTAAAACAGGAAATCCCATATTTGCCATC GGCATATATTAAGTATATTAGCAGTTCTAATTACAAACTAC ACCCTGAAGAGCAGATTTCCTTCACTTCAGAAGAAATCGA GGGCAAGCCCACCCACTACAAATTGAAAATTTTAGTCAGT GGTAGTTGGAAGGATACTATCCTTTATGAGATCCCCTTACT GTCCCTAATATCAGAAGCGTATTTTAAATTTGTTGACATCG ACTGGGACTACGAAAACCAATTAGAACAAGCTGAGAAGAA GGCGGAAACTTTGTTTGATAATGGTATTAGATTCAGTGAAT TTGGTACAAGACGTCGTAGATCTCTGAAGGCTCAAGATCTA ATTATGCAAGGAATCATGAAAGCTGTGAACGGTAACCCAG ACAGAAACAAATCGCTATTATTAGGCACATCAAATATTTTA TTTGCCAAGAAATATGGAGTCAAGCCAATCGGTACTGTGG CTCACGAGTGGGTTATGGGAGTCGCTTCTATTAGTGAAGAT TATTTGCATGCCAATAAAAATGCAATGGATTGTTGGATCAA TACTTTTGGTGCAAAAAATGCTGGTTTAGCATTAACGGATA CTTTTGGAACTGATGACTTTTTAAAATCATTCCGTCCACCA TATTCTGATGCTTACGTCGGTGTTAGACAAGATTCTGGAGA CCCAGTTGAGTATACCAAAAAGATTTCCCACCATTACCATG ACGTGTTGAAATTGCCTAAATTCTCGAAGATTATCTGTTAT TCCGATTCTTTGAACGTCGAAAAGGCAATAACTTACTCCCA TGCAGCTAAAGAGAATGGAATGCTAGCCACATTCGGTATT GGCACAAACTTTACTAATGATTTTCGTAAGAAGTCAGAACC CCAGGTTAAAAGTGAGCCGTTAAACATCGTTATCAAACTAT TAGAAGTAAATGGTAATCACGCTATCAAAATTTCTGATAAC TTAGGTAAAAATATGGGAGATCCTGCCACTGTGAAGAGAG TGAAAGAGGAATTGGGATATACTGAACGAAGTTGGAGTGG TGATAACGAAGCGCACAGATGGACCTAA | 1 |
| primer | Kim1808 | CAGCAGTATCTTTTTAGACCTAATCTTGACAAAACCACCCA TGAACCACAC | 67 |
| | Kim1809 | CAAAAGAGACTTTATCACTGGTTCTGACATTATTGATATAG TGTTTAAGCGAATG | 68 |
| | Kim1806 | TCCAGTGTAGTAGCCATGTTTTAGAGCTAGAAATAGCAAG | 69 |
| | Kim1807 | TGGCTACTACACTGGAAGTGATCATTTATCTTTCACTGCG | 70 |
| | Kim549 | GCATCCTTTGCCTCCGTTC | 71 |
| | Kim1811 | CAAAGTTTCCGCCTTCTTCTCAG | 72 |
| gRNA; pRS42H-*NPT1*ₚ | | ACTTCCAGTGTAGTAGCCATCGG | 73 |

### 3-2. Evaluation of NMN Productivity of NPT1-Overexpressing Mutant Strain

To evaluate the NMN productivity according to *NPT1* overexpression, the NMN production amount of the *NPT1*-overexpressing mutant strain RE03 was compared with those of RE01-1 from Example 1 and RE02-1 from Example 2.

Each strain was inoculated into 10 mL of YPD medium (containing 10 g/L yeast extract, 20 g/L peptone, and 20 g/L glucose) and pre-cultured at 30°C and 250 rpm for 24 hours. The pre-culture was then inoculated at 0.5% (v/v) into fresh YPD medium and main-cultured at 30°C and 250 rpm for 11 hours. After the main culture, 50 mg of properly grown cells were disrupted using Y-PER^{™} yeast protein extraction reagent (Thermo Scientific) and protease inhibitor and used for evaluation of NMN productivity.

The cell lysate was reacted in a reaction buffer containing 20% (v/v) crude extract per 0.6 mL reaction mixture, 50 mM Tris-HCl (pH 7.5), 10 mM MgCl₂, 10 mM ATP, 40 mM NAM, and 5 mM PRPP. After a 12-hour reaction, the mixture was filtered through a 0.45 µm filter, and the NMN production amount was analyzed using HPLC (Agilent Technologies 1260 Infinity, Agilent Technologies) equipped with a column (Supelcosil^{™} LC-18) and a UV detector (260 nm).

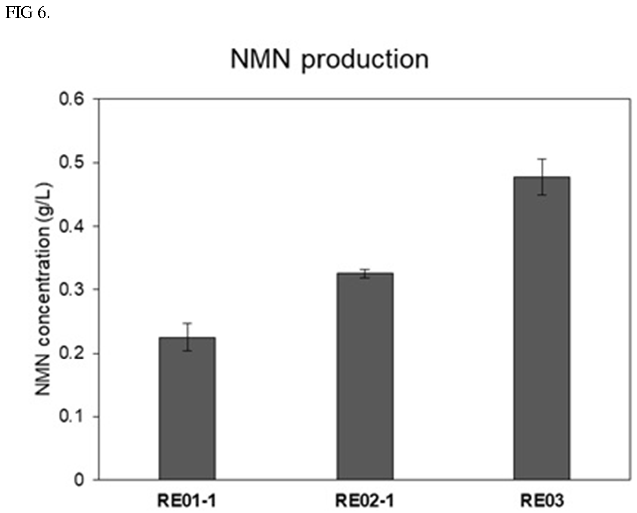

As a result, referring to FIG. 6, the NPT1-overexpressing mutant strain RE03 showed the highest NMN production, suggesting that NAM, which is structurally similar to NA, can be converted into NMN by NPT1.

### Example 4. Overexpression of Endogenous Gene NPT1

To determine whether NPT produces NMN using NAM as a substrate in the absence of NAMPT, NPT1 was overexpressed by replacing the promoter of the endogenous NPT1 in *Saccharomyces cerevisiae* with a strong promoter or by increasing the copy number.

### 4-1. Construction of NPT1 Promoter-Enhanced Mutant Strain

Prior to constructing a mutant strain overexpressing *NPT1, PNC1* was removed using *Saccharomyces cerevisiae* DY2 as the parent strain.

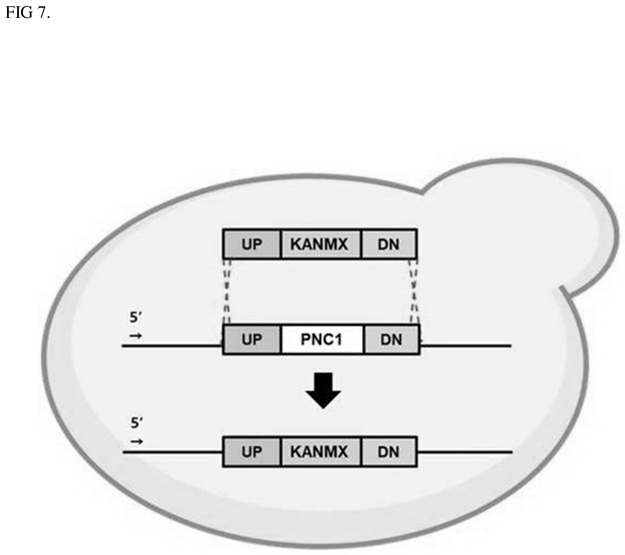

*PNC1* is an endogenous gene that converts NAM into NA; the *PNC1* gene was removed by replacing it with the kanamycin antibiotic resistance gene (*KANMX*) (see FIG. 7). Genomic DNA was extracted from a *PNC1* knockout strain obtained from Yeast Deletion Clones (Invitrogen) as a template, and the specific kanamycin antibiotic resistance gene was amplified by PCR using primers Kim1683 and Kim1686 and Q5^{®} High-Fidelity DNA Polymerase. PCR was carried out at 98°C for 30 s, 30 cycles of (98°C for 5 s, 60°C for 10 s, and 72°C for 1 min 30 s), and 72°C for 2 min. The constructed kanamycin antibiotic resistance gene was introduced into strain DY2 by the LiAc transformation method, and the selection of the target strain was confirmed by colony PCR using primers Kim1685 and Kim284, which yielded a band of 1.5 kb; the resulting strain was designated DY2 *pnc1::KanMX*.

The primers used are shown in Table 6 below.

**[Table 6]**

| Name | | **Sequence (5'-3')** | **SEQ ID NO.** |
|---|---|---|---|
| primer | Kim1683 | TTTCCTCTTTCCCTACGATC | 74 |
| | Kim1686 | TTGTGAATGAGAAGTCGTAG | 75 |
| | Kim1685 | GGATGGAGCTGTTTATGGATGG | 76 |
| | Kim284 | AATTTAATCGCGGCCTCG | 77 |

Subsequently, a mutant strain was constructed from the DY2 *pnc1::KanMX* strain by overexpressing *PRS1* in the same manner as in Example 2-1, while also overexpressing *NPT1* via the CCW12 promoter in the same manner as in Example 3-1; this strain was designated YJ02.

### 4-2. Construction of NPT1-2-Copy-Expressing Mutant Strain

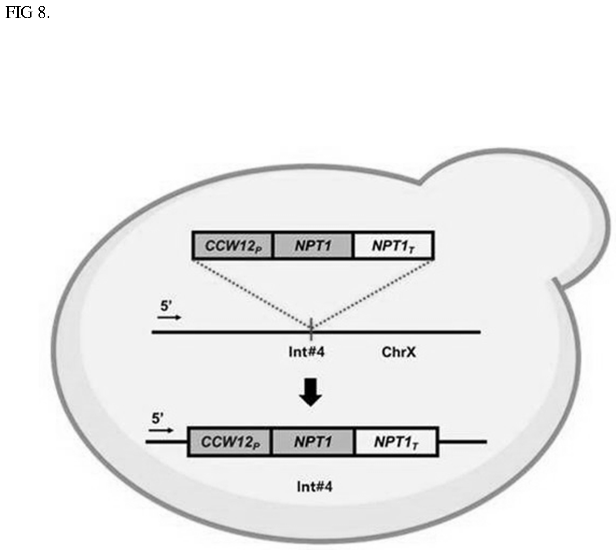

A mutant strain expressing 2 copies of *NPT1* in *Saccharomyces cerevisiae* was constructed (see FIG. 8).

The plasmid pRS42H-int#4 was constructed in the same manner as in Example 3-1.

Using YJ02 from Example 4-1 as a template, the *NPT1* expression cassette was amplified by PCR using primers Kim1822 and Kim1823 and Q5^{®} High-Fidelity DNA Polymerase (initial denaturation: 98°C for 30 s; 30 cycles of 98°C for 5 s, 62°C for 10 s, and 72°C for 55 s; final extension: 72°C for 2 min). The plasmid pRS42H-int#4 and the *NPT1* expression cassette were introduced into YJ02, which had been constructed in Example 4-1, by the LiAc transformation method. The selection of the target strain was confirmed by colony PCR using primers Kim322 and Kim323, which yielded a band of 2.5 kb, and the resulting strain was designated YJ03.

The primers and gRNA used are shown in Table 7 below.

**[Table 7]**

| Name | | **Sequence (5'-3')** | **SEQ ID NO.** |
|---|---|---|---|
| primer | Kim1822 | CATTTCTTTTCCTCGGGCAGAGAAACTCGCAGGCA ACTTGCCACCCATGAACCACAC | 78 |
| | Kim1823 | GTAACGGATCCCTCTGTGAGGGCCGATTATGCAGG CCTAGATTGCCCTGCAAAAGCAG | 79 |
| | Kim322 | GCGCATCTATTTGCCGTC | 80 |
| | Kim323 | TCACGACACACCTCACTG | 81 |
| gRNA; | | CTCTCGAAGTGGTCACGTGCGGG | 82 |
| pPRS42H-int#4 | | | |

### 4-3. Evaluation of NMN-Productivity of NPT1-Overexpressing Mutant Strains

The NMN production amounts of YJ02 from Example 4-1 and YJ03 from Example 4-2 were compared.

Each strain was inoculated into 10 mL of YPD medium (containing 10 g/L yeast extract, 20 g/L peptone, and 20 g/L glucose) and pre-cultured at 30°C and 250 rpm for 24 hours. The pre-culture was then inoculated at 0.5% (v/v) into YPX medium (containing 10 g/L yeast extract, 20 g/L peptone, and 20 g/L xylose) and main-cultured at 30°C and 250 rpm for 16 hours. After the main culture, 50 mg of properly grown cells were disrupted using Y-PER^{™} yeast protein extraction reagent (Thermo Scientific) and protease inhibitor and used for evaluation of NMN productivity.

The cell lysate was reacted in a reaction buffer containing 20% (v/v) crude extract per 0.6 mL reaction mixture, 50 mM Tris-HCl (pH 7.5), 10 mM MgCl₂, 10 mM ATP, 40 mM NAM, and 5 mM PRPP. After a 3-hour reaction, the mixture was filtered through a 0.45 µm filter, and the NMN production amount was analyzed using HPLC (Agilent Technologies 1260 Infinity, Agilent Technologies) equipped with a column (Supelcosil^{™} LC-18) and a UV detector (260 nm).

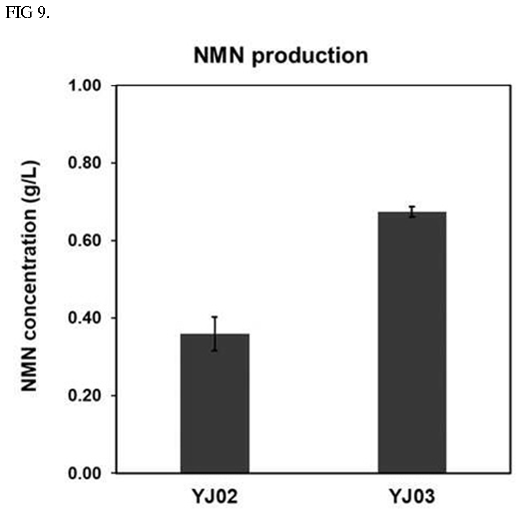

As a result, referring to FIG. 9, strain YJ03, by expressing 2 copies of NPT1, showed increased NMN production compared to strain YJ02 due to the increased copy number.

### Example 5. Verification of Essential Mutation Elements for High NMN Production

### 5-1. Construction of PNC1-Removed and/or NPT1-Overexpressing Mutant Strains

DY2 *pnc1::KanMX*, constructed in Example 4-1, was used as the *PNC1-*removed mutant strain.

The *NPT1*-overexpressing mutant strain was constructed using the same gRNA plasmid and *NPT1* expression cassette as in Example 4-2. The plasmid pRS42H-int#4 and the *NPT1* expression cassette were introduced into *Saccharomyces cerevisiae* D452-2 by the LiAc transformation method. The selection of the target strain was confirmed in the same manner as in Example 4-2, and the resulting strain was designated DH01.

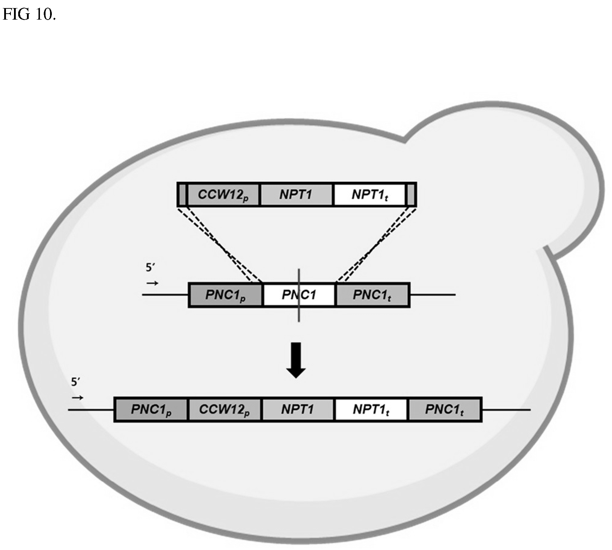

In order to construct a mutant strain in which *PNC1* was removed and *NPT1* was overexpressed, *PNC1* was removed and an *NPT1* expression cassette was introduced into the corresponding locus (see FIG. 10).

The same gRNA plasmid as in Example 1-1 was used.

Using RE03 from Example 3-1 as a template, the *NPT1* expression cassette was amplified by PCR using primers Kim1936 and Kim1937 and Q5^{®} High-Fidelity DNA Polymerase (initial denaturation: 98°C for 30 s; 30 cycles of 98°C for 5 s, 60°C for 10 s, and 72°C for 65 s; final extension: 72°C for 2 min). The plasmid pRS42H-*PNC1* and the *NPT1* expression cassette were introduced into *Saccharomyces cerevisiae* D452-2 by the LiAc transformation method. The selection of the target strain was confirmed by colony PCR using primers Kim549 and Kim1938, which yielded a band of 1.79 kb, and the resulting strain was designated D452-2 *pnc1::NPT1*.

The primers and gRNA used are shown in Table 8 below.

**[Table 8]**

| Name | | **Sequence (5'-3')** | **SEQ ID NO.** |
|---|---|---|---|
| primer | Kim1936 | TTGTTAGAAAGAATAAAATACAGTACAAAACCACCC ATGAACCACAC | 83 |
| | Kim1937 | TCATCAGGTTGAAGAAGTATTATTCAGCTCTTGCCCT GCAAAAGCAG | 84 |
| | Kim549 | GCATCCTTTGCCTCCGTTC | 85 |
| | Kim1938 | GCAAGCCACCCTAGTTC | 86 |
| gRNA; pRS42H-*PNC1* | | AGAAAAGCATCATACAGACGAGG | 87 |

### 5-2. Evaluation of NMN Productivity of PNC1-Removed and/or NPT1-Overexpressing Mutant Strains

To evaluate the NMN productivity according to removal of *PNC1* and/or overexpression of *NPT1*, the NMN production amounts of the *PNC1*-removed mutant strain (DY2 *pnc1::KanMX*) from Example 4-1, the *NPT1*-overexpressing mutant strain (DH01) from Example 5-1, and the *PNC1*-removed and *NPT1*-overexpressing mutant strain (D452-2 *pnc1::NPT1*) from Example 5-1 were compared.

Each strain was inoculated into 10 mL of YPD medium (containing 10 g/L yeast extract, 20 g/L peptone, and 20 g/L glucose) and pre-cultured at 30°C and 250 rpm for 24 hours. The pre-culture was then inoculated at 0.5% (v/v) into fresh YPD medium and main-cultured at 30°C and 250 rpm for 11 hours. After the main culture, 50 mg of properly grown cells were disrupted using Y-PER^{™} yeast protein extraction reagent (Thermo Scientific) and protease inhibitor and used for evaluation of NMN productivity.

The cell lysate was reacted in a reaction buffer containing 20% (v/v) crude extract per 0.6 mL reaction mixture, 50 mM Tris-HCl (pH 7.5), 10 mM MgCl₂, 10 mM ATP, 40 mM NAM, and 5 mM PRPP. After a 3-hour reaction, the mixture was filtered through a 0.45 µm filter, and the NMN production amount was analyzed using HPLC (Agilent Technologies 1260 Infinity, Agilent Technologies) equipped with a column (Supelcosil^{™} LC-18) and a UV detector (260 nm).

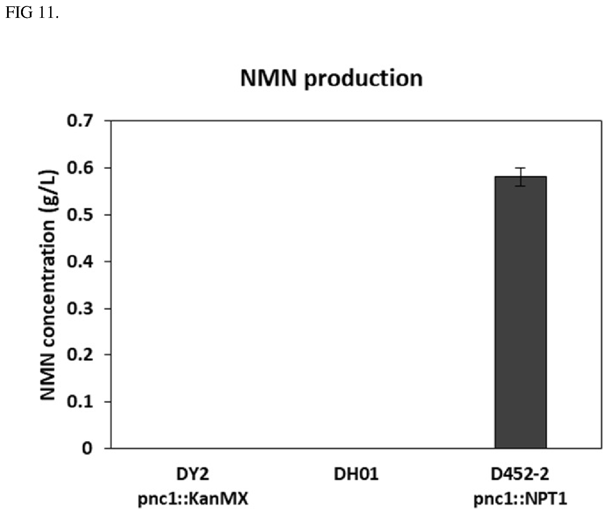

As a result, referring to FIG. 11, NMN was not produced in either the strain in which PNC1 alone was removed (DY2 *pnc1::KanMX*) or the strain into which the *NPT1* expression cassette alone was introduced (DH01), whereas the strain in which *PNC1* was removed and the *NPT1* expression cassette was introduced (D452-2 *pnc1::NPT1*) produced NMN at a high concentration. These results suggest that removal of *PNC1* and overexpression of *NPT1* are essential mutation elements for NMN production.

### Example 6. Removal of Endogenous Gene POF1

### 6-1. Construction of POF1-Removed Mutant Strain

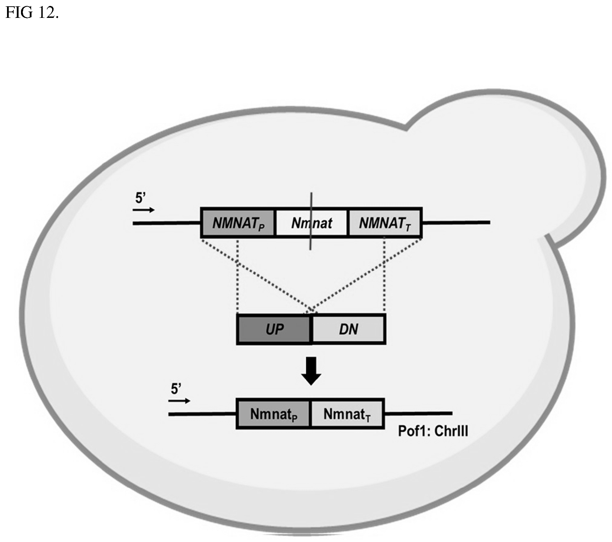

In order to prevent the conversion of NMN to NAD⁺ in *Saccharomyces cerevisiae*, a mutant strain was constructed in which *POF1*, one of the genes encoding NMNAT, was removed (see FIG. 12).

The plasmid pRS42H-*POF1* was constructed using primers Kim1766 and Kim1767 in the same manner as in Example 4-1.

Using *Saccharomyces cerevisiae* as a template and primers Kim1784 and Kim1785, respectively, a *POF1* fragment was prepared by PCR using Q5^{®} High-Fidelity DNA Polymerase (initial denaturation: 98°C for 30 s; 30 cycles of 98°C for 5 s, 61°C for 10 s, and 72°C for 10 s; final extension: 72°C for 2 min).

The constructed fragment was introduced into the YJ02 strain from Example 4-1 by the LiAc transformation method. The selection of the target strain was confirmed by colony PCR using primers Kim1798 and Kim1799, which yielded a band of the *POF1* (0.24 kb) size, and the resulting strain was designated YJ02 POF1Δ.

The *POF1* primers and gRNA used are shown in Table 9 below.

**[Table 9]**

| Name | | **Sequence (5'-3')** | **SEQ ID NO.** |
|---|---|---|---|
| *POF1* | | ATGAAGAAGACGTTCGAGCAGTTTCGAAAAAGCAAT TTACTATTTCAGGTTCTCAAAGGACCCCAGCATCTAG AATGTCAGAAGTTATTGTCCTTGATTCTTCATTCAA TCCACCACATCTGGCCCATTTTCAACTACTATCGCAG ACTATTAAAAACTTCAAATTGAAGGACACCCGTTCG CATGTTTTATTACTGTTAGCGGTGAATAATGCAGATA AGTTGCCTAAGCCGGCATCTTTTCCAACTCGTCTGGA AATGATGTGCTTATTCGCTGACTACCTTCAGGAGAAG CTCCCCCAATCTGTAGTATCTGTCGGGTTGACTGTTT TCTCGAAATTCATCGACAAGGACAAAATATTACATG AGCAATTTGTTAAAGGATGCAGTGCAGATATAGGCT ACTTAGTTGGTTTTGATACAATTGCTAGGATCTTTGA TGAAAAATATTATCATCCTTTAAAAATCAGTGATGTA ATGGAGAGCTTCATGTCGGGATCTCAATTATATTGCT TGGCGAGAGGCGATTGCCATCTCAGTGCTGAATCGC AACTAAGATACGCCAGTGACATCCTTGAGGGAAAAT TCGAACCGGTAATACCAAGAGAATGGGGCGCTAGGA TTCATGTTATGCAAAATGATTATCCAGCATTAAGAAA TGTTTCATCATCCGAGATTAGGAACAAACTGAAGAA TGGGCAAGTGGAGAGTTTGAAAGACGAGTTGCCATT GTGCATATACGATTATTTGATCAATAATAAGACAAT ATTTGATTGA | 47 |
| primer | Kim1784 | GTTATCCCAATTAATGATAATTTCATTATCACTCATT TCAGATCTTTGTACC | 88 |
| | Kim1785 | GTTAAACAGGATGGTACAATCATAGAAAAAGGTAC AAAGATCTGAAATGAG | 89 |
| | Kim1766 | CAGATACTACAGATTGGGTTTTAGAGCTAGAAATAG CAAG | 90 |
| | Kim1767 | CAATCTGTAGTATCTGTCGGATCATTTATCTTTCACT GCG | 91 |
| | Kim1798 | TGAATGCAAGAGGTAAACCAGC | 92 |
| | Kim1799 | GGACAAACCCATCAAATGGAAG | 93 |
| gRNA; pRS42H-*POF1* | | CGACAGATACTACAGATTGGGGG | 94 |

### 6-2. Evaluation of NMN Productivity of POF1-Removed Mutant Strain

To evaluate the NMN productivity according to removal of *POF1*, the NMN production amounts of YJ02 from Example 4-1, YJ03 from Example 4-2, and the *POF1-*removed mutant strain YJ02 POF1Δ from Example 6-1 were compared.

Each strain was inoculated into 10 mL of YPD medium (containing 10 g/L yeast extract, 20 g/L peptone, and 20 g/L glucose) and pre-cultured at 30°C and 250 rpm for 24 hours. The pre-culture was then inoculated at 0.5% (v/v) into fresh YPD medium and main-cultured at 30°C and 250 rpm for 11 hours. After the main culture, 50 mg of properly grown cells were disrupted using Y-PER^{™} yeast protein extraction reagent (Thermo Scientific) and protease inhibitor and used for evaluation of NMN productivity.

The cell lysate was reacted in a reaction buffer containing 20% (v/v) crude extract per 0.6 mL reaction mixture, 50 mM Tris-HCl (pH 7.5), 10 mM MgCl₂, 10 mM ATP, 40 mM NAM, and 5 mM PRPP. After a 3-hour reaction, the mixture was filtered through a 0.45 µm filter, and the NMN production amount was analyzed using HPLC (Agilent Technologies 1260 Infinity, Agilent Technologies) equipped with a column (Supelcosil^{™} LC-18) and a UV detector (260 nm).

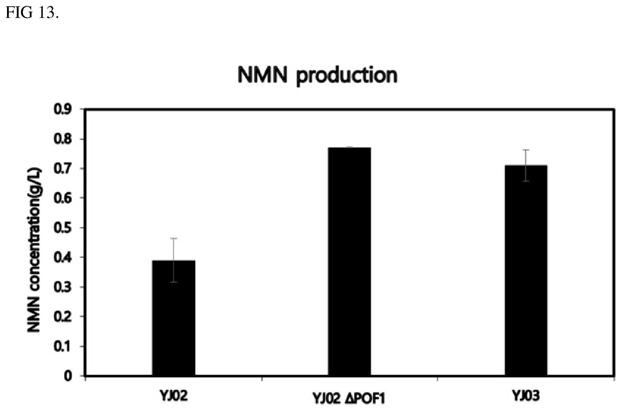

As a result, referring to FIG. 13, the *POF1*-removed mutant strain (YJ02 POF1Δ) showed increased NMN production compared to strain YJ02, in which *POF1* was not removed, and exhibited an NMN production level similar to that of strain YJ03, in which the *NPT1* copy number was increased. These results suggest that inhibiting the conversion of NMN to NAD⁺ can contribute to an increase in NMN production.

The present invention has been described above with a focus on its preferred embodiments. A person having ordinary skill in the art to which the present invention pertains will understand that the present invention can be implemented in modified forms without departing from the essential characteristics of the present invention. Therefore, the disclosed embodiments should be considered from an illustrative point of view rather than a limiting point of view. The scope of the present invention is shown in the claims rather than the foregoing description, and all differences within the equivalent scope thereof should be construed as being included in the present invention.

## Claims

1. A mutant microorganism having enhanced nicotinamide mononucleotide productivity, wherein the activity of nicotinate phosphoribosyltransferase is enhanced.

2. The mutant microorganism according to claim 1, wherein the nicotinate phosphoribosyltransferase is derived from one or more selected from the group consisting of *Saccharomyces cerevisiae, Schizosaccharomyces pombe, Methanosarcina mazei, Methanosarcina acetivorans, Desulforapulum autotrophicum, Methanococcoides burtonii, Chromohalobacter salexigens, Arabidopsis thaliana, Dictyostelium discoideum, Ciona intestinalis, Drosophila melanogaster, Ciona savignyi, Bacillus subtilis, Caenorhabditis elegans, Mycobacterium tuberculosis, Candida glabrata or Nakaseomyces glabratus, Kluyveromyces lactis, Eremothecium gossypii, Debaryomyces hansenii, Candida albicans, Yarrowia lipolytica, Aspergillus fumigatus, Neurospora crassa, Chaetomium globosum, Emericella nidulans, Aspergillus oryzae, Gibberella moniliformis, Cryptococcus neoformans,* and *Ustilago maydis*.

3. The mutant microorganism according to claim 1, wherein the enhancement of the activity of nicotinate phosphoribosyltransferase is achieved by a nucleotide modification of the gene encoding nicotinate phosphoribosyltransferase, an increase in the copy number of the gene, a modification of the regulatory region of the gene, introduction of a foreign gene, or a combination thereof.

4. The mutant microorganism according to claim 1, wherein the mutant microorganism has a further attenuated activity of nicotinamidase.

5. The mutant microorganism according to claim 1, wherein the mutant microorganism has a further attenuated activity of nicotinamide mononucleotide adenylyltransferase.

6. The mutant microorganism according to claim 1, wherein the mutant microorganism has a further enhanced activity of nicotinamide phosphoribosyltransferase.

7. The mutant microorganism according to claim 1, wherein the mutant microorganism has a further enhanced activity of phosphoribosylpyrophosphate synthase.

8. The mutant microorganism according to claim 1, wherein the mutant microorganism is of the genus *Saccharomyces*.

9. The mutant microorganism according to claim 1, wherein the mutant microorganism is *Saccharomyces cerevisiae*.

10. A method for producing nicotinamide mononucleotide, comprising step of: culturing the mutant microorganism of claim 1 in a medium; and recovering nicotinamide mononucleotide from the mutant microorganism or from the medium in which the mutant microorganism has been cultured.
